# EUROPEAN PATENT APPLICATION

(11) **EP 3 659 593 A1**
(43) Date of publication of application: **03.06.2020**
(21) Application number: 18209641.2
(22) Date of filing: 30.11.2018
(51) Int. Cl.: A61K 31/167, A61K 31/198, A61K 31/4015, A61K 31/4155, A61K 31/44, A61K 31/47, A61K 31/519, A61K 31/09, A61K 31/52, A61P 25/14, A61K 9/00

(54) **TRMT2A INHIBITORS FOR USE IN THE TREATMENT OF POLYGLUTAMINE DISEASES**

(71) Applicant: Forschungszentrum Jülich GmbH/ Abteilung RP-PT, 52428 Jülich (DE)
(72) Inventor: ROSSETTI,Guilia., 52062 Aachen (DE); MARGREITER, Michael Alois., 51065 Köln (DE); NIESSING, Dierk., 89075 Ulm (DE); DAVYDOVA, Elena., 80797 München (DE); WITZENBERGER, Monika., 80933 München (DE); SCHULZ, Jörg., 52074 Aachen (DE); VOIGT, Aaron., 52064 Aachen (DE); SHAH, Nadim Joni., 52428 Jülich (DE)
(74) Representative: Greiner, Elisabeth

(57) **Abstract**

The present invention relates to inhibitors of the enzyme TRMT2A and medical uses thereof. More particularly, the present invention relates to the treatment and the prevention of polyglutamine diseases using TRMT2A inhibitors.

## Description

### FIELD OF THE INVENTION

The present invention relates to inhibitors of the enzyme TRMT2A and medical uses thereof. More particularly, the present invention relates to TRMT2A inhibitors for use in the treatment and/or the prevention of polyglutamine diseases.

### BACKGROUND OF THE INVENTION

Polyglutamine (polyQ) diseases like Huntington's disease (HD) and several spinocerebellar ataxias (SCAs) represent a heterogenic group of neurodegenerative disorders (Fan, Ho et al. 2014). Apart of the dominant inheritance, polyQ diseases have only one commonality, an expanded stretch of CAG repeats within the coding region of the disease-linked gene. This CAG repeat translates into an undisrupted polyQ stretch of various length in the respective disease-causing protein (Landles and Bates 2004).

The polyQ stretch itself is causative for the disease and there is a direct correlation between the length of the undisrupted polyQ stretch, age of onset and disease severity. The longer the polyQ stretch, the earlier the disease onset and the more severe is the disease progression. This feature allows a very accurate prediction of disease-onset, progression and death already in newborn affected individuals. In the disease situation, the polyQ harboring protein is usually cleaved, leaving the polyQ stretch intact. PolyQ peptides are aggregation prone and assemble via an oligomeric state into fibrils. Finally, accumulated polyQ peptides then form cytoplasmic and nuclear aggregates, the hallmark of polyQ diseases. Some of these aggregated polyQ structures (most likely oligomers and fibrils) are believed to contribute to the neuronal decline in disease situation.

Until now, most of the polyQ diseases are incurable and current treatment options merely aim at mitigating disease-associated symptoms, such as movement and emotional problems. To date, FDA approved therapeutics are only available to treat symptoms of patients with Huntington's disease. At present, there are several ongoing clinical trials, with the majority focusing on patients already suffering from HD. The approaches range from mindfulness-based cognitive therapy, physical activity coaching intervention to dietary supplementation (Brogueira Rodrigues and Wild 2018). In particular promising results from the first-in-human phase 1b/2a trial of IONIS-HTTRx, the antisense oligonucledotide designed to lower HTT in a dose-dependent manner, received recognition beyond the medical community (NCT02519036) (Labbadia and Morimoto 2013) (Colpo, Stimming et al. 2017) (Takeuchi and Nagai 2017) (Southwell, Skotte et al. 2012).

An apparent drawback of this therapeutic strategy is the requirement of administration via repeated lumbar punctures, which could result in lower patient compliance. Furthermore, it is presently only investigated for HD patients.

Although each polyQ disease (e.g. Huntington's disease or Machado-Joseph disease) itself has a low incidence, in sum the group of polyQ diseases represents the third most common class of neurodegenerative disorders, ranging after Alzheimer's and Parkinson's disease (not taking the auto-immune disorder Multiple sclerosis into account).

Thus, there is a need in the art for improved, at best neuroprotective, treatment options of polyQ diseases in order to suppress or delay the occurrence of disease associated symptoms, prolong the asymptomatic period and slow down disease progression.

### SUMMARY OF THE INVENTION

The present invention provides an inhibitor of the enzyme TRMT2A. Also provided is an inhibitor of the enzyme TRMT2A for use in therapy. In one embodiment, the inhibitor of the enzyme TRMT2A is for use in treating and/or preventing a polyglutamine disease in a subject. The polyglutamine disease may be Huntington's disease, a spinocerebellar ataxia, dentatorubral pallidoluysian atrophy or spinal and bulbar muscular atrophy. In a preferred embodiment, the polyglutamine disease is Huntington's disease.

Also provided is a pharmaceutical composition comprising a TRMT2A inhibitor and a pharmaceutically acceptable excipient. The composition may comprise one or more additional therapeutically active agents. The present invention also relates to a method for inhibiting TRMT2A, wherein the method comprises contacting a cell *in vitro* with an inhibitor of TRMT2A.

### BRIEF DESCRIPTION OF THE DRAWINGS

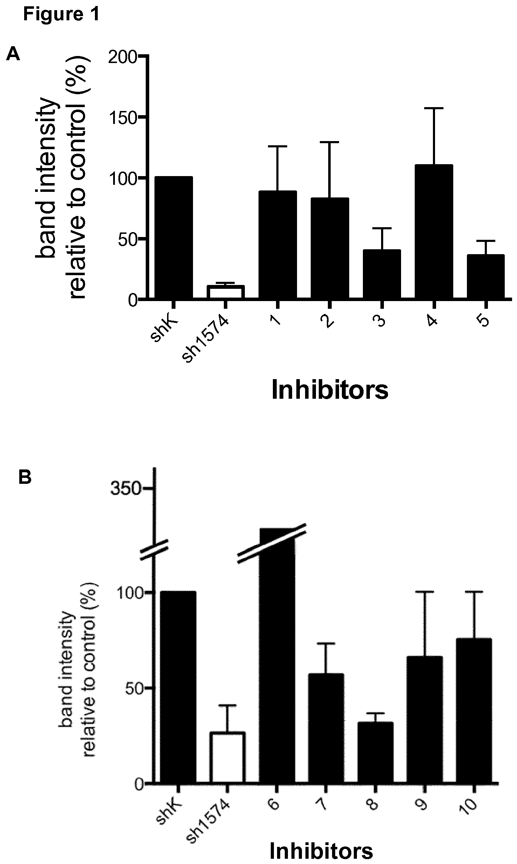

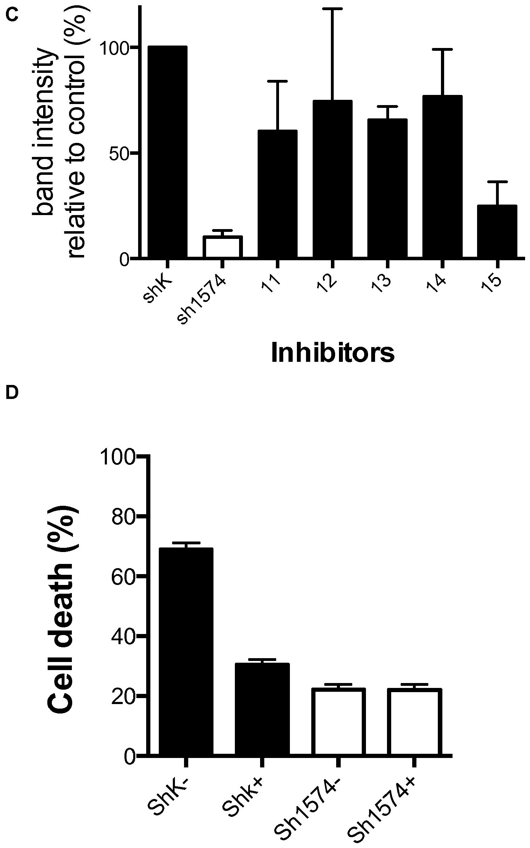

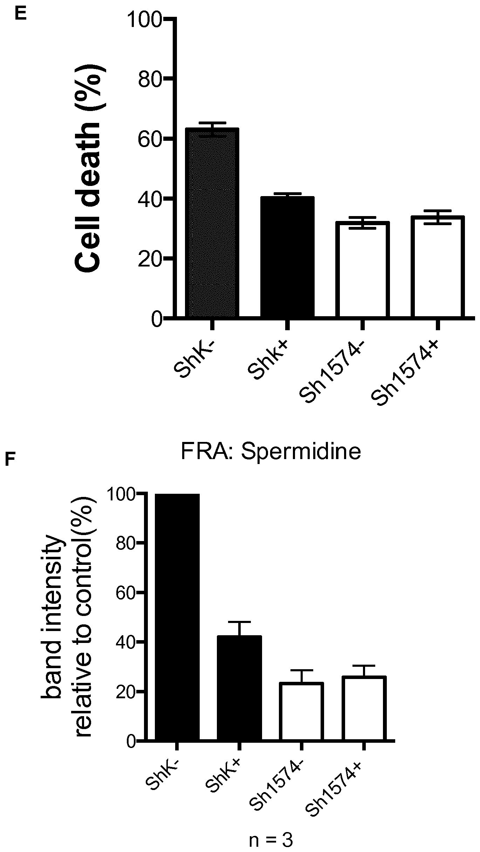

- **Figure 1**:: Cell Death Assay: HEK293T cells transfected with PolyQ103 (huntingtin (HTT) harbouring a polyQ expansion of 103 glutamines fused to GFP was cloned into the pRC/CMV vector (Invitrogen)) were treated with different TRMT2A inhibitors and the amount of dead cells was assessed after 48 hours post transfection; ShK = cells with stable expression of a scrambled hair pin (control); Sh1574 = cells with stable expression of a TRMT2A-specific hair pin (TRMT2A-silenced cells); shown are mean values for n = 9 biological replicates, with 4 technical replicates each. Biological replicates were specified as independent wells.
Dot Blot Assay: HEK293T cells transfected with PolyQ103 (see above) were treated with different TRMT2A inhibitors and the amount of PolyQ aggregates was assessed.
1A: Dot blot analysis of inhibitors 1-5 (for inhibitor structures see table 1).
1B: Dot blot analysis of inhibitors 6-10 (for inhibitor structures see table 1).
1C: Dot blot analysis of inhibitors 11-15 (for inhibitor structures see table 1).
1D: Cell death assay of inhibitor 15 (spermidine 100 µM); manual cell count.
1E: Cell death assay of inhibitor 15 (spermidine 100 µM); automatic cell count.
1F: Filter Retardation Assay
- **Figure 2:**: 2A: Cell death assay of inhibitors 16-24 (for inhibitor structures see table 1)
2B: Cell death assay of inhibitors 25-32 (for inhibitor structures see table 1)
- **Figure 3:**: 3A: Dot blot analysis of inhibitor 16
3B: Quantification of 3A
- **Figure 4:**: 4A: Dot blot analysis of inhibitor 17
4B: Quantification of 4A
- **Figure 5:**: 5A: Dot blot analysis of inhibitor 19
5B: Quantification of 5A
- **Figure 6:**: 6A: Dot blot analysis of inhibitor 21
6B: Quantification of 6A
- **Figure 7:**: 7A: Dot blot analysis of inhibitor 22
7B: Quantification of 7A
- **Figure 8:**: 8A: Dot blot analysis of inhibitor 24
8B: Quantification of 8A
- **Figure 9:**: 9A: Dot blot analysis of inhibitor 25
9B: Quantification of 9A
- **Figure 10**:: Cartoon drawing of the RNA-binding domain (aa 69-147) of TRMT2A solved at 2.02 Å resolution.

### DETAILED DESCRIPTION OF THE INVENTION

To gain more insight in the molecular mechanisms involved in the neuronal decline upon polyQ expression, the inventors of the present invention performed an unbiased, large-scale screen in *Drosophila.* This screen was set to identify genes that when silenced by RNAi modify polyQ-induced toxicity. In this screen, it was found that silencing of *CG3808,* coding the fly ortholog of human TRMT2A strongly suppressed polyQ-induced toxicity and aggregation, the key pathological findings of polyQ diseases. TRMT2A is a tRNA (uracil-5-) -methyltransferase converting uridine to 5-methyl uridine at position 54 of tRNAs. Methylation of tRNAs at position 54 presumably enhances correct binding of respective amino acid/aminoacyl-tRNA-synthetase. The effects caused by silencing TRMT2A were verified in yeast (*Saccharomyces cerevisiae*) (Nordlund, Johansson et al. 2000), human cell line (*Human embryonic kidney,* HEK cells) and fibroblasts derived from Huntington's disease patients indicating that the protective effect of TRMT2A silencing on polyQ-induced toxicity and aggregation is conserved between various species (Zoghbi and Orr 2000).

Of note, in none of these organisms/cells TRMT2A-silencing had an effect on viability and even homozygous TRMT2A knockout mice did not display any obvious pathological phenotype at 1 year of age. In all analyzed organisms/cells, the absence of TRMT2A reduced polyQ aggregation and toxicity. The absence of TRMT2A, however, did not impair vital functions in all organisms/cells analyzed.

Presumably, a lack of TRMT2A enzymatic function causes an error prone translation, finally causing the insertion of a non-glutamine amino acid in the otherwise undisrupted polyQ stretch. Such a modification is known to ameliorate polyQ-induced toxicity. Inhibition of TRMT2A's methyl-transferase activity was identified as a key point for potential therapy of polyQ diseases.

The inventors further identified numerous inhibitors of TRMT2A based on *in silico* modeling. These inhibitors have been tested in HEK293T cells with regard to polyQ-induced toxicity and polyQ aggregation. In these assays, several inhibitors have been shown to reduce polyQ-aggregation and toxicity. In support of these efforts, a crystal structure of the TRMT2A RNA-binding domain has been solved at 2.02 Å resolution.

Accordingly, the TRMT2A inhibitors disclosed herein offer a treatment approach for neurodegenerative diseases, such as polyQ diseases, allowing treatment and/or prevention of these diseases.

### Definitions

Unless defined otherwise, all technical and scientific terms used herein have the same meaning as commonly understood by one of ordinary skill in the art (e.g., in cell culture, molecular genetics, nucleic acid chemistry, hybridization techniques and biochemistry).

The term "polyglutamine disease" or "polyQ disease" relates to a group of neurodegenerative disorders caused by an expanded stretch of CAG repeats within the coding region of a disease-linked gene. This CAG repeat translates into an undisrupted polyQ stretch of various length in the respective disease-causing protein. Examples for polyQ diseases are Huntington's disease, spinocerebellar ataxia 1, 2, 3 (also termed Kennedy's disease), 6, 7 and 17, dentatorubral pallidoluysian atrophy (DRPLA) and spinal and bulbar muscular atrophy (SBMA). For the most common variant of polyQ diseases, Huntington's disease, a prevalence of 2.71:100,000 world-wide has been assessed in 2012 (Pringsheim, Wiltshire et al. 2012).

The term "Huntington's disease" refers to a hereditary, progressive neurodegenerative disorder characterized by abnormal involuntary movements, behavioral disturbance, cognitive dysfunction, and psychiatric disease (Bates 2005). The disease is caused by a CAG trinucleotide repeat expansion in exon 1 of the huntingtin (HTT) gene giving rise to an elongated polyQ tract at the amino terminus of the translated huntingtin (HTT) protein (Colpo, Stimming et al. 2017). The mechanisms of cell toxicity of the mutant HTT possibly involve multiple pathways such as abnormal protein aggregation, mitochondrial dysfunction and excitotoxiticy, among others. Current treatment options are limited to suppressing chorea (the involuntary, irregular movements of the arms and legs) that accompanies the disease and mood altering aspect (Labbadia and Morimoto 2013).

"TRMT2A" relates to tRNA methyltransferase 2 homolog A. Eponymous for these proteins is *Saccharomyces cerevisiae* TRM2. TRM2 converts uridine to 5-methyl uridine (m⁵U) at position 54 (Sharma, Yang et al. 2013) (Nordlund, Johansson et al. 2000). Although methylation of a tRNA varies from species to species for different amino acid acceptors (Rodríguez-Hernández, Bhaskaran et al. 2010), the methylation of m⁵U at position 54 is conserved and found from yeast to man. There are two scTRM2 homologs in human, termed as *h*TRMT2A and *h*TRMT2B (Towns and Begley 2012) (Dunin-Horkawicz, Czerwoniec et al. 2006) with *h*TRMT2A being the real TRM2 ortholog. Yeast deficient for the *TRM2* lack the U₅₄ methyl group in their tRNAs, but consistent with data from flies and mice, do not show any obvious pathological phenotypes (Nordlund, Johansson et al. 2000). The function of aminoacyl-tRNA-synthetases (aaRSs) consists of loading tRNAs with their cognate specific amino acid (Guo and Schimmel 2013). Originally, it has been assumed that for each amino acid one aminoacyl-tRNA-synthetase exisits, that catalyzes a two-step reaction of charging the tRNA with its specific amino acid (Beebe, Mock et al. 2008). Thus, it was assumed that a complete set of twenty aminoacyl-tRNA-synthetases (aaRSs) is required for survival. However, later analyses revealed that some bacteria, chloroplast and mitochondria lack certain aaRS proteins (Schön, Kannangara et al. 1988) (Sheppard, Yuan et al. 2008).

These organisms/organelles do not discriminate between glutamine (Gln) and glutamate (Glu). Instead, they use the so-called transamidation pathway where the tRNA^{Gln} is loaded with Glu. This mischarge is later compensated by two consecutive enzymatic reactions catalyzing the exchange of Glu-tRNA^{Gln} to Gln-tRNA^{Gln} (Ibba, Curnow et al. 1997) (Horiuchi, Harpel et al. 2001) (Schön and Söll 1988). Thus, in the context of evolution, the discrimination between Glu and Gln seems to be relatively new.

In a TRMT2A deficient background, it is expected that especially the translation of the long and undisrupted glutamine stretch in polyQ peptides is error prone. Presence of a non-glutamine amino acid in an otherwise undisrupted polyQ stretch is known to diminish its toxicity (Menon, Nethisinghe et al. 2013).

The term "TRMT2A inhibitor" or "inhibitor of TRMT2A" relates to a compound which inhibits the methyl-transferase activity of the enzyme TRMT2A partially or completely. Hence, a TRMT2A inhibitor partially or completely blocks TRMT2A-mediated tRNA methylation. For example, a TRMT2A inhibitor inhibits methyl-transferase activity of the enzyme TRMT2A by at least 20%. A TRMT2A inhibitor may inhibit methyl-transferase activity of the enzyme TRMT2A by at least30 %. A TRMT2A inhibitor may inhibit methyl-transferase activity of the enzyme TRMT2A by at least 40%. A TRMT2A inhibitor may inhibit methyl-transferase activity of the enzyme TRMT2A by at least 50%. A TRMT2A inhibitor may inhibit methyl-transferase activity of the enzyme TRMT2A by at least 60%. A TRMT2A inhibitor may inhibit methyl-transferase activity of the enzyme TRMT2A by at least 70%. In a preferred embodiment, a TRMT2A inhibitor inhibits methyl-transferase activity of the enzyme TRMT2A by at least 30%. Inhibition of TRMT2A activity may be measured directly by measuring uridine to 5-methyl uridine conversion. The amount of 5-methyl uridine can be measured in the supernatant of a cell culture or in a cell-free enzymatic assay. Alternatively, TRMT2A inhibition may be measured indirectly by measuring the decrease in polyQ aggregation in cells.

### TRMT2A inhibitors / Medical uses

In one aspect, the present invention provides inhibitors of the enzyme TRMT2A. In another aspect, the present invention relates to inhibitors of the enzyme TRMT2A for use in therapy.

In one embodiment, the TRMT2A inhibitors of the present invention are for use in the treatment of a neurodegenerative disease in a subject. In one embodiment, the TRMT2A inhibitors of the present invention are for use in the prevention of a neurodegenerative disease in a subject.

In a further embodiment, the TRMT2A inhibitors of the present invention are for use in the treatment of a polyQ disease in a subject. In a further embodiment, the TRMT2A inhibitors of the present invention are for use in the prevention of a polyQ disease in a subject.

Also provided is a method for treating a polyQ disease, wherein the method comprises administering a therapeutically effective amount of a TRMT2A inhibitor to a patient in need thereof.

PolyQ diseases include Huntington's disease, spinocerebellar ataxias, dentatorubral pallidoluysian atrophy or spinal and bulbar muscular atrophy. In one embodiment, the polyQ disease is Huntington's disease.

PolyQ diseases can be diagnosed before the subject shows any symptoms of the disease (prodromal stage). According to their dominant inheritance, p olyQ diseases usually appear in individuals with a familiar background of the respective disease. *De novo* appearance is low (<10% in case of HD). PolyQ diseases are commonly diagnosed by genetic testing. The inhibitors of the present invention may be administered to subjects carrying the disease-linked mutant allele at prodromal stage as well as to symptomatic patients in disease stage. In one embodiment, the subject is a human patient.

Advantageously, the inhibitors of the present invention may already be administered to a subject known to carry the disease-linked mutant allele in prodromal stages (in which the subject does not yet have any symptoms) thereby enabling pre-symptomatic, neuroprotective treatment. Administration of a TRMT2A inhibitor to a subject in prodromal disease stage may delay onset of the polyQ disease symptoms. Accordingly, administration of a TRMT2A inhibitor to a subject at a pre-symptomatic disease stage may stop or delay the onset of the polyQ disease. Administration of a TRMT2A inhibitor to a subject at a pre-symptomatic disease stage may prolong the asymptomatic period of the polyQ disease. As symptoms in polyQ diseases are a direct result of neurodegeneration/neuronal dysfunction, administration of a TRMT2A inhibitor to a subject at a pre-symptomatic disease stage may delay or prevent neuronal decline in the subject suffering from a polyglutamine disease. Administration of a TRMT2A inhibitor to a subject at a pre-symptomatic disease stage may delay or suppress the occurrence of polyglutamine disease associated symptoms. Administration of a TRMT2A inhibitor to a subject at a pre-symptomatic disease stage may delay or prevent neuronal decline in the subject suffering from a polyglutamine disease.

The inhibitors of the present invention may also be administered to a subject having a polyglutamine disease in order to mitigate or suppress the symptoms of the polyQ disease at a symptomatic disease stage. Administration of a TRMT2A inhibitor to a subject at a symptomatic disease stage may prolong the time between polyglutamine disease onset and death. Administration of a TRMT2A inhibitor to a subject at a symptomatic disease stage may stop polyglutamine disease progression. Administration of a TRMT2A inhibitor to a subject at a symptomatic disease stage may reverse disease progression. Administration of a TRMT2A inhibitor to a subject at a symptomatic disease stage may reduce the severity of symptoms of a polyglutamine disease.

In other embodiments, the inhibitors of the present invention are administered in combination with one or more further therapeutic agents, such as Tetrabenazine or its derivatives.

In one embodiment, the TRMT2A inhibitor has formula (I): 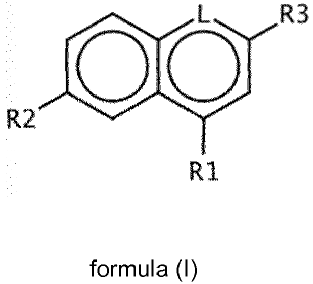 wherein
L is C or N;
R1 is OH or [(2-{[(benzyloxy)carbonyl] amino}ethoxy) carbonyl]amino; R2 is H or benzyl; and
R3 is H or CO2.

In one embodiment, the inhibitor has the following formula: 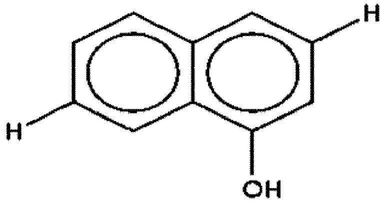 (naphthol).

In one embodiment, the inhibitor has the following formula: 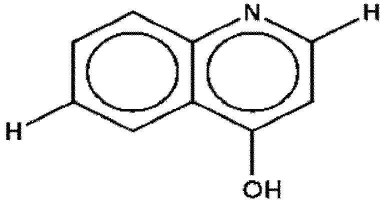 (4-hydroxyquinoline).

In one embodiment, the inhibitor has the following formula: 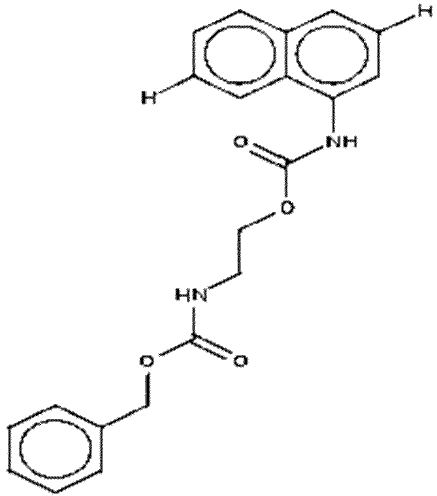 (benzyl N-(2-{[(naphthalen-1-yl)carbamoyl]oxy}ethyl)carbamate).

In one embodiment, the inhibitor has the following formula: 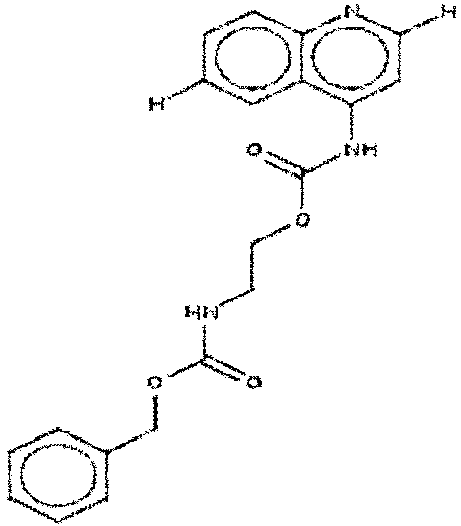 (benzyl N-(2-{[(quinolin-4-yl)carbamoyl]oxy}ethyl)carbamate).

In one embodiment, the inhibitor has the following formula: 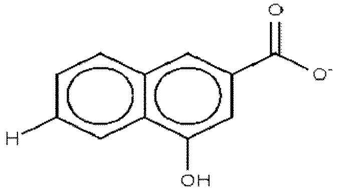 (4-hydroxynaphthalene-2-carboxylate).

In one embodiment, the inhibitor has the following formula: 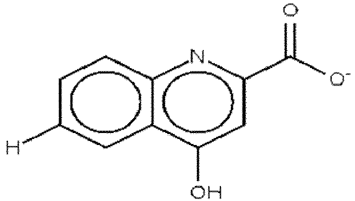 (kynurenate).

In one embodiment, the inhibitor has the following formula: 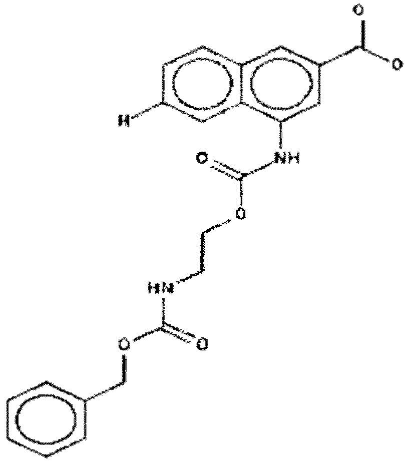 (4-{[(2-{[(benzyloxy)carbonyl]amino}ethoxy)carbonyl]amino}naphthalene-2-carboxylate).

In one embodiment, the inhibitor has the following formula: 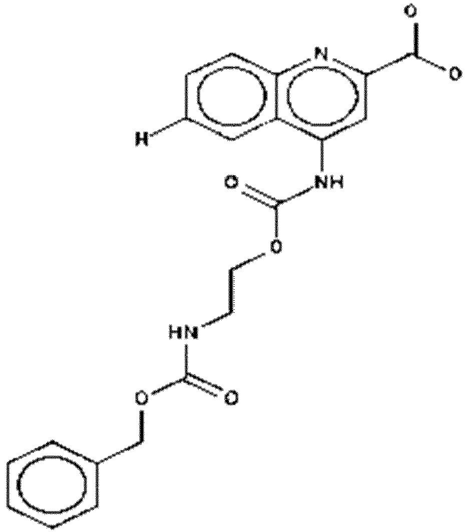 (4-{[(2-{[(benzyloxy)carbonyl]amino}ethoxy)carbonyl]amino}quinoline-2-carboxylate).

In one embodiment, the inhibitor has the following formula: 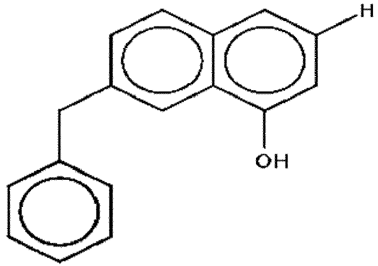 (7-benzylnaphthalen-1-ol).

In one embodiment, the inhibitor has the following formula: 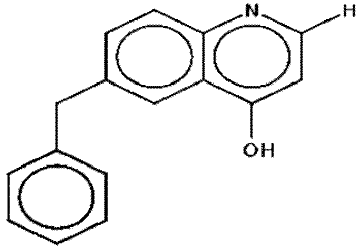 (6-benzylquinolin-4-ol).

In one embodiment, the inhibitor has the following formula: 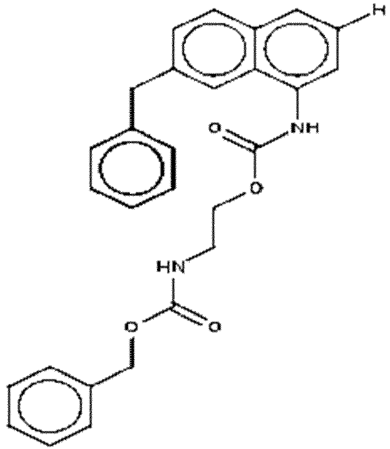 (benzyl N-(2-{[(7-benzylnaphthalen-1-yl)carbamoyl]oxy}ethyl)carbamate).

In one embodiment, the inhibitor has the following formula: 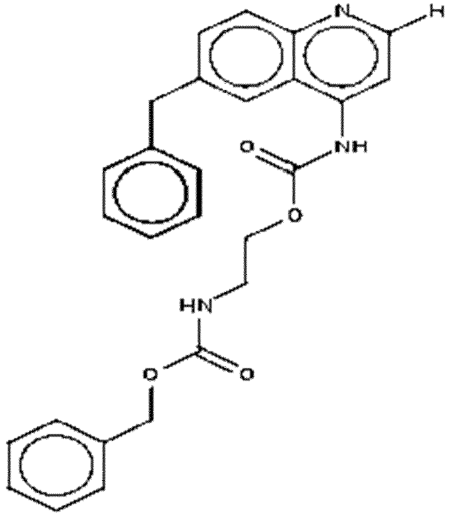 (benzyl N-(2-{[(6-benzylquinolin-4-yl)carbamoyl]oxy}ethyl)carbamate).

In one embodiment, the inhibitor has the following formula: 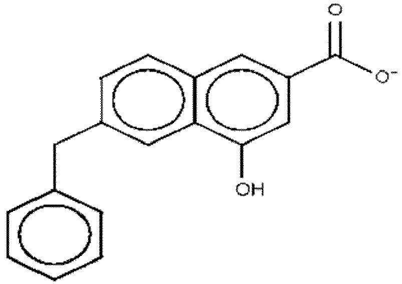 (6-benzyl-4-hydroxynaphthalene-2-carboxylate).

In one embodiment, the inhibitor has the following formula: 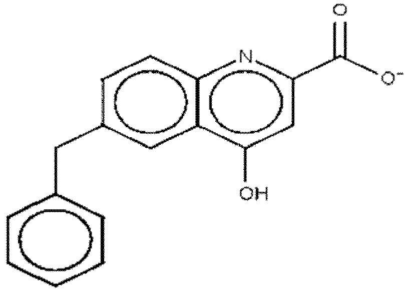 (6-benzyl-4-hydroxyquinoline-2-carboxylate).

In one embodiment, the inhibitor has the following formula: 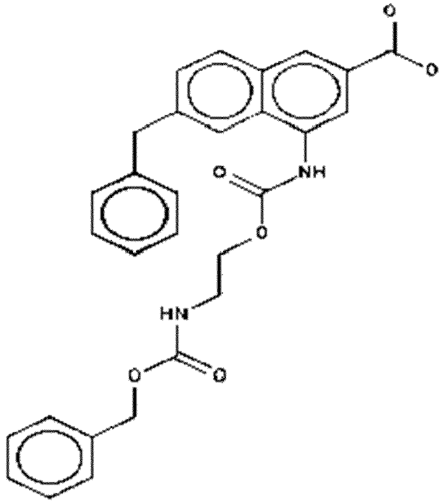 (6-benzyl-4-{[(2-{[(benzyloxy)carbonyl]amino}ethoxy)carbonyl]amino}naphthalene-2-carboxylate).

In one embodiment, the inhibitor has the following formula: 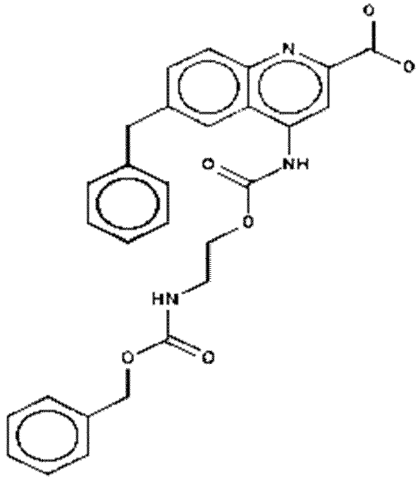 (6-benzyl-4-{[(2-{[(benzyloxy)carbonyl]amino}ethoxy)carbonyl]amino}quinoline-2-carboxylate).

In one embodiment, the TRMT2A inhibitor of the invention has formula (II): 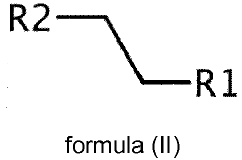 wherein
R1 is H, CH3, NH2, SCH3, ethylsulfanyl or (3-aminopropyl)amino; and
R2 is amino(carboxy)methyl or CH2CHNH2R3, wherein R3 is H or COOH.

In one embodiment, the inhibitor has the following formula: 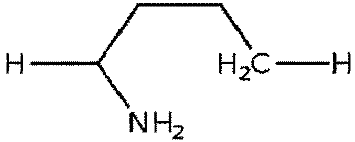 (butylamine).

In one embodiment, the inhibitor has the following formula: 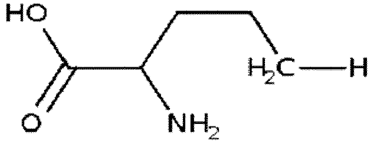 (Nva).

In one embodiment, the inhibitor has the following formula: 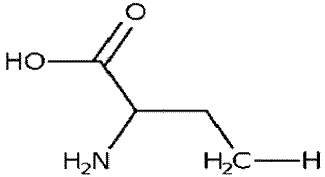 (Abu).

In one embodiment, the inhibitor has the following formula: 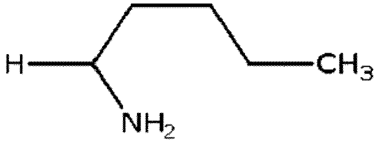 (amylamine).

In one embodiment, the inhibitor has the following formula: 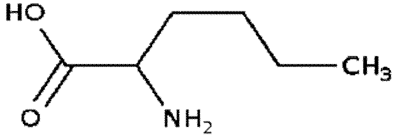 (norleucine).

In one embodiment, the inhibitor has the following formula: 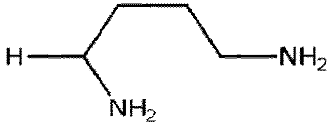 (putrescine).

In one embodiment, the inhibitor has the following formula: 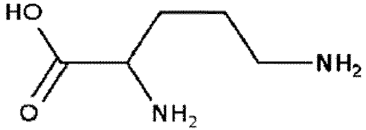 (ornithine).

In one embodiment, the inhibitor has the following formula: 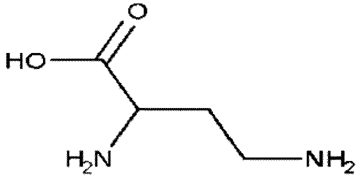 (2,4-diaminobutanoic acid).

In one embodiment, the inhibitor has the following formula: 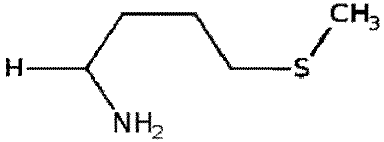 (4-(methylsulfanyl)butan-1-amine).

In one embodiment, the inhibitor has the following formula: 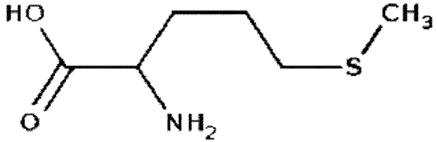 (homomethionine).

In one embodiment, the inhibitor has the following formula: 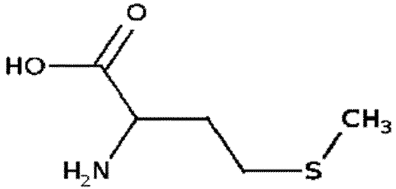 (methionine).

In one embodiment, the inhibitor has the following formula: 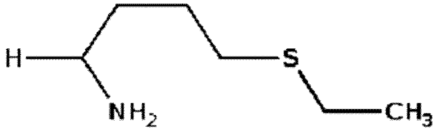 (4-(ethylsulfanyl)butan-1-amine).

In one embodiment, the inhibitor has the following formula: 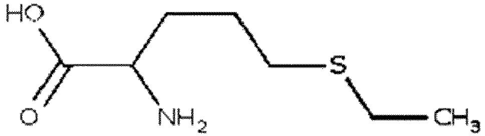 (2-amino-5-(ethylsulfanyl)pentanoic acid).

In one embodiment, the inhibitor has the following formula: 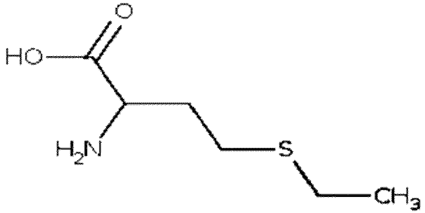 ((D)-ethionine).

In one embodiment, the inhibitor has the following formula: 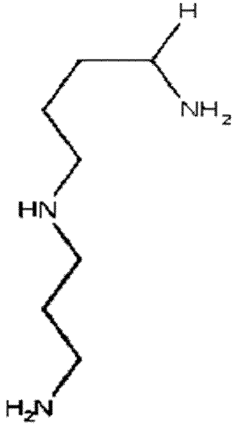 (spermidine).

In one embodiment, the inhibitor has the following formula: 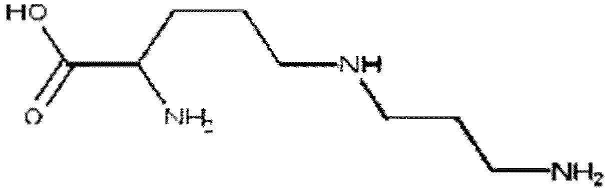 (2-amino-5-[(3-aminopropyl)amino]pentanoic acid).

In one embodiment, the inhibitor has the following formula: 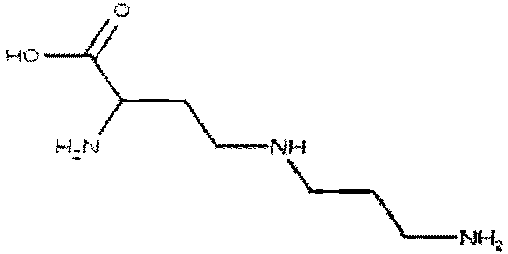 (2-amino-4-[(3-aminopropyl)amino]butanoic acid).

In one embodiment, the TRMT2A inhibitor of the invention has formula (III): 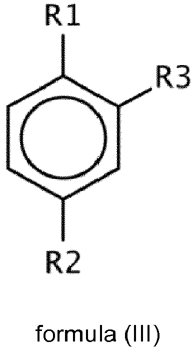 wherein
R1 is selected from the group consisting of piperidine-1-carbonyl, 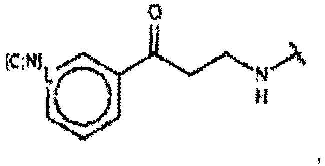 5-bromo-2-(carboxylatomethoxy)benzoyl, and 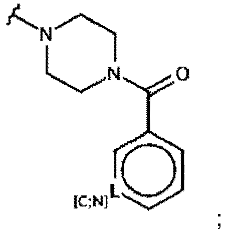 R2 is selected from the group consisting of hydrogen, carboxylatomethoxy, pyridine-4-carbonyl, and [(5-oxo-2,5-dihydro-1H-1,2,4-triazol-3-yl)methyl]amino; and
R3 is selected from the group consisting of hydrogen, and Cl.

In one embodiment, the inhibitor has the following formula: 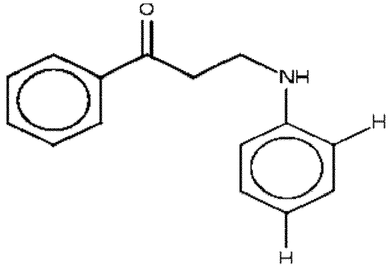 (1-phenyl-3-(phenylamino)propan-1-one).

In one embodiment, the inhibitor has the following formula: 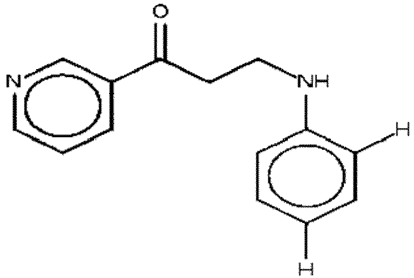 (3-(phenylamino)-1-(pyridin-3-yl)propan-1-one).

In one embodiment, the inhibitor has the following formula: 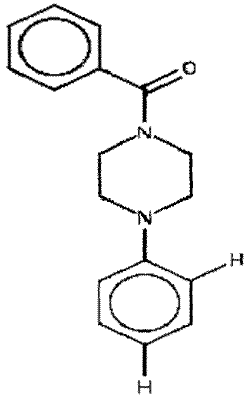 (1-benzoyl-4-phenylpiperazine).

In one embodiment, the inhibitor has the following formula: 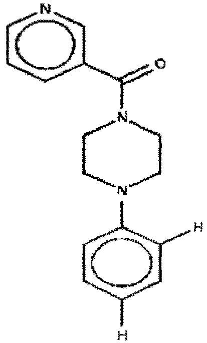 (1-phenyl-4-(pyridine-3-carbonyl)piperazine).

In one embodiment, the inhibitor has the following formula: 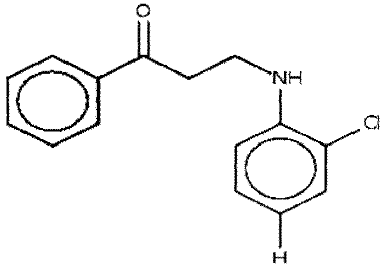 (3-[(2-chlorophenyl)amino]-1-phenylpropan-1-one).

In one embodiment, the inhibitor has the following formula: 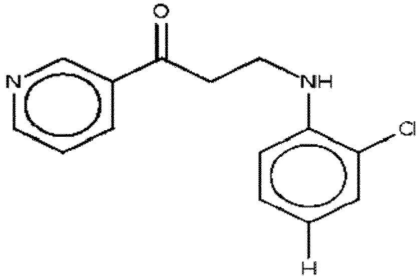 (3-[(2-chlorophenyl)amino]-1-(pyridin-3-yl)propan-1-one).

In one embodiment, the inhibitor has the following formula: 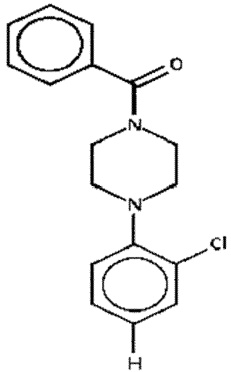 (1-benzoyl-4-(2-chlorophenyl)piperazine).

In one embodiment, the inhibitor has the following formula: 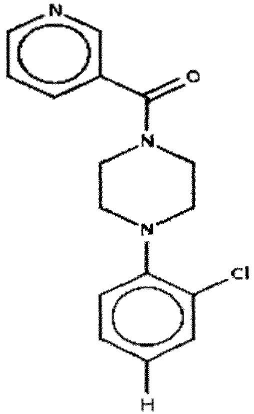 (1-(2-chlorophenyl)-4-(pyridine-3-carbonyl)piperazine).

In one embodiment, the inhibitor has the following formula: 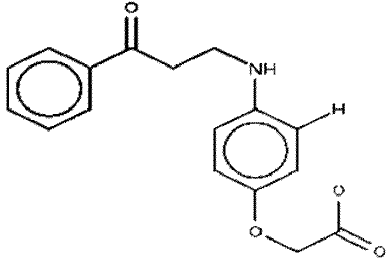 (2-{4-[(3-oxo-3-phenylpropyl)amino]phenoxy}acetate).

In one embodiment, the inhibitor has the following formula: 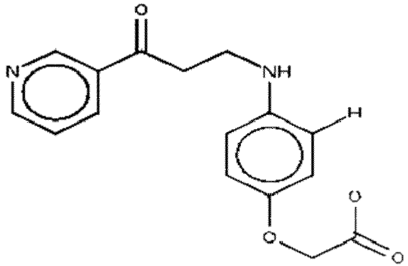 (2-(4-{[3-oxo-3-(pyridin-3-yl)propyl]amino}phenoxy)acetate).

In one embodiment, the inhibitor has the following formula: 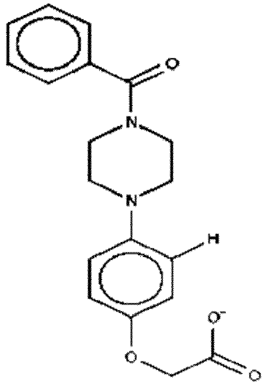 (2-[4-(4-benzoylpiperazin-1-yl)phenoxy]acetate).

In one embodiment, the inhibitor has the following formula: 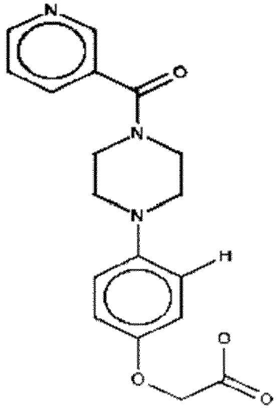 (2-{4-[4-(pyridine-3-carbonyl)piperazin-1-yl]phenoxy}acetate).

In one embodiment, the inhibitor has the following formula: 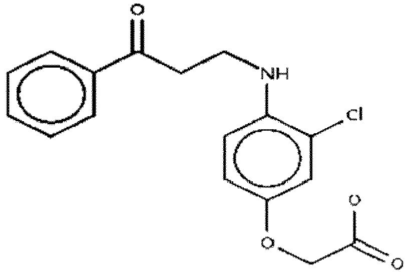 (2-{3-chloro-4-[(3-oxo-3-phenylpropyl)amino]phenoxy}acetate).

In one embodiment, the inhibitor has the following formula: 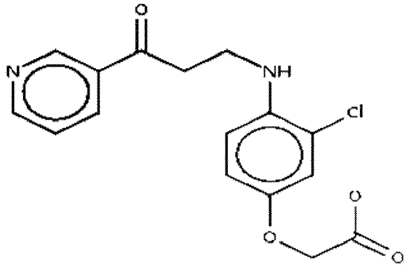 (2-(3-chloro-4-{[3-oxo-3-(pyridin-3-yl)propyl]amino}phenoxy)acetate).

In one embodiment, the inhibitor has the following formula: 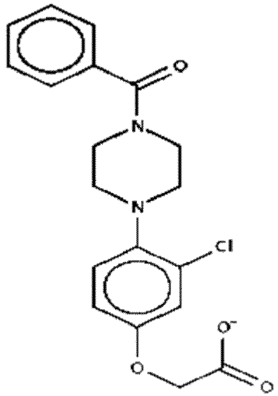 (2-[4-(4-benzoylpiperazin-1-yl)-3-chlorophenoxy]acetate).

In one embodiment, the inhibitor has the following formula: 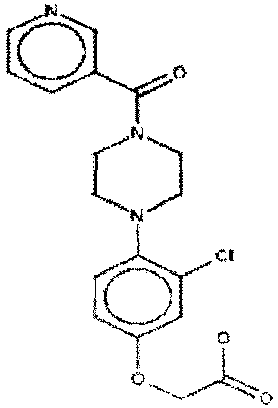 (2-{3-chloro-4-[4-(pyridine-3-carbonyl)piperazin-1-yl]phenoxy}acetate).

In one embodiment, the inhibitor has the following formula: 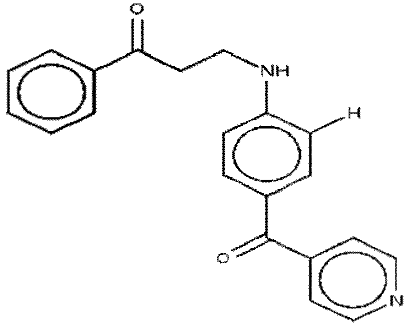 (1-phenyl-3-{[4-(pyridine-4-carbonyl)phenyl]amino}propan1-one).

In one embodiment, the inhibitor has the following formula: 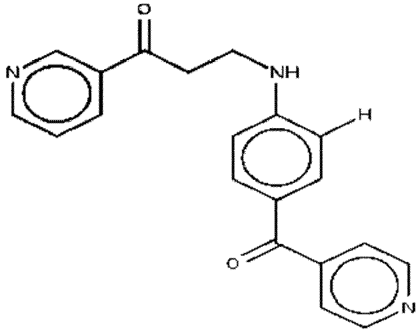 (1-(pyridin-3-yl)-3-{[4-(pyridine-4-carbonyl)phenyl]amino}propan-1-one).

In one embodiment, the inhibitor has the following formula: 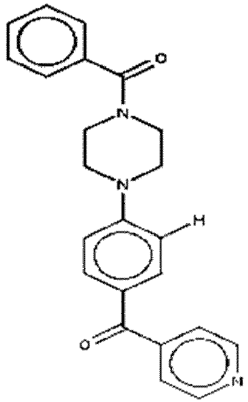 (1-benzoyl-4-[4-(pyridine-4-carbonyl)phenyl]piperazine).

In one embodiment, the inhibitor has the following formula: 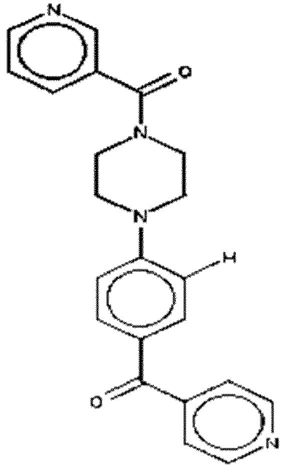 (1-(pyridine-3-carbonyl)-4-[4-(pyridine-4-carbonyl)phenyl]piperazine).

In one embodiment, the inhibitor has the following formula: 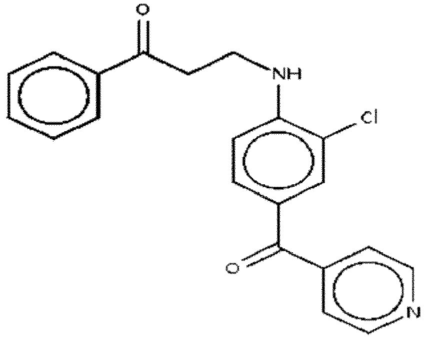 (3-{[2-chloro-4-(pyridine-4-carbonyl)phenyl]amino}-1-phenylpropan-1-one).

In one embodiment, the inhibitor has the following formula: 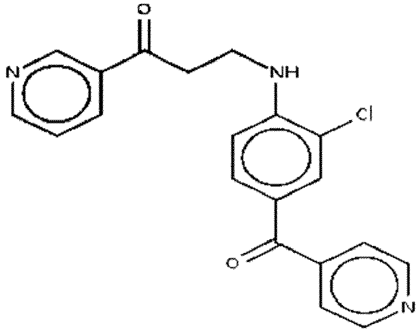 (3-{[2-chloro-4-(pyridine-4-carbonyl)phenyl]amino}-1-(pyridin-3-yl)propan-1-one).

In one embodiment, the inhibitor has the following formula: 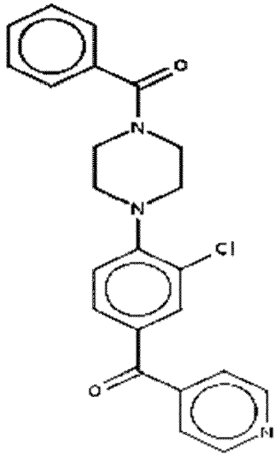 (1-benzoyl-4-[2-chloro-4-(pyridine-4-carbonyl)phenyl]piperazine).

In one embodiment, the inhibitor has the following formula: 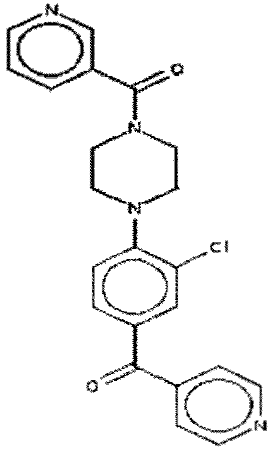 (1-[2-chloro-4-(pyridine-4-carbonyl)phenyl]-4-(pyridine-3-carbonyl)piperazine).

In one embodiment, the inhibitor has the following formula: 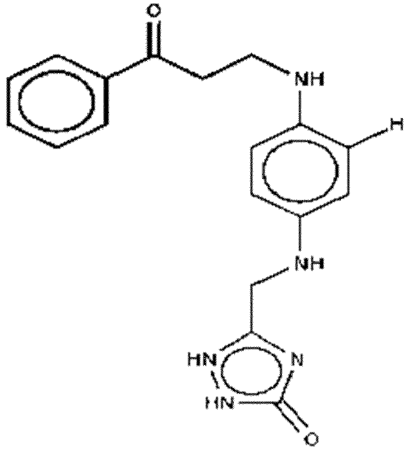 (5-[({4-[(3-oxo-3-phenylpropyl)amino]phenyl}amino)methyl]-1,2-dihydro-1,2,4-triazol-3-one).

In one embodiment, the inhibitor has the following formula: 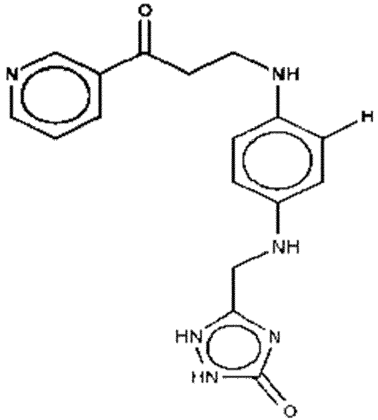 (5-{[(4-{[3-oxo-3-(pyridin-3-yl)propyl]amino}phenyl)amino]methyl}-1,2-dihydro-1,2,4-triazol-3-one).

In one embodiment, the inhibitor has the following formula: 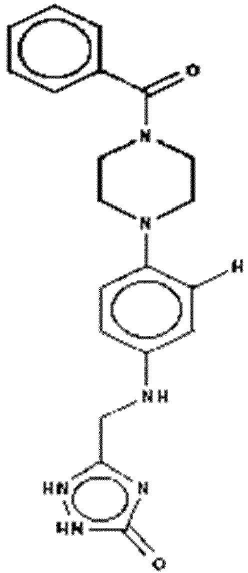 (5-({[4-(4-benzoylpiperazin-1-yl)phenyl]amino}methyl)-1,2-dihydro-1,2,4-triazol-3-one).

In one embodiment, the inhibitor has the following formula: 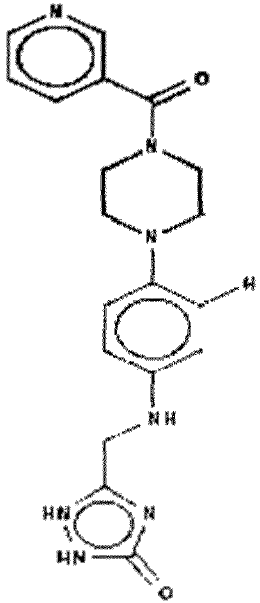 (5-[({4-[4-(pyridine-3-carbonyl)piperazin-1-yl]phenyl}amino)methyl]-1,2-dihydro-1,2,4-triazol-3-one).

In one embodiment, the inhibitor has the following formula: 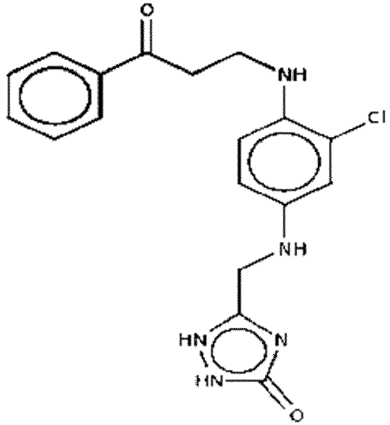 5-[({3-chloro-4-[(3-oxo-3-phenylpropyl)amino]phenyl}amino)methyl]-1,2-dihydro-1,2,4-triazol-3-one

In one embodiment, the inhibitor has the following formula: 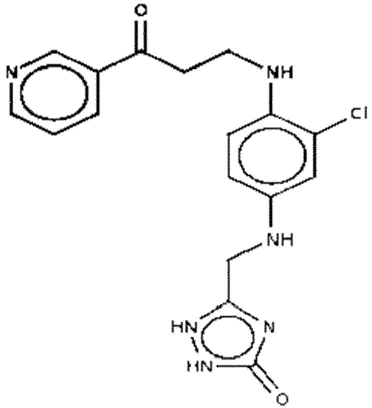 (5-{[(3-chloro-4-{[3-oxo-3-(pyridin-3-yl)propyl]amino}phenyl)amino]methyl}-1,2-dihydro-1,2,4-triazol-3-one).

In one embodiment, the inhibitor has the following formula: 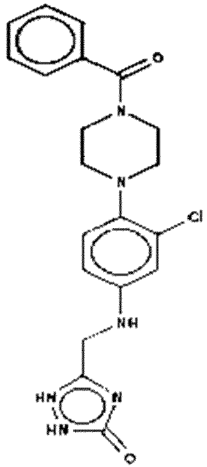 (5-({[4-(4-benzoylpiperazin-1-yl)-3-chlorophenyl]amino}methyl)-1,2-dihydro-1,2,4-triazol-3-one).

In one embodiment, the inhibitor has the following formula: 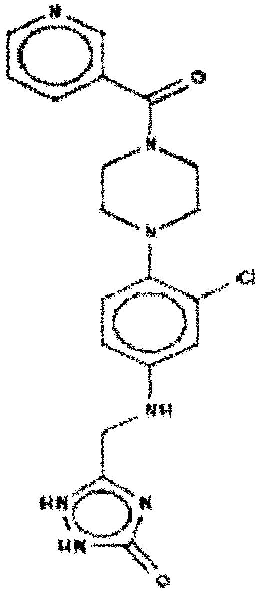 (5-[({3-chloro-4-[4-(pyridine-3-carbonyl)piperazin-1-yl]phenyl}amino)methyl]-1,2-dihydro-1,2,4-triazol-3-one).

In one embodiment, the inhibitor has the following formula: 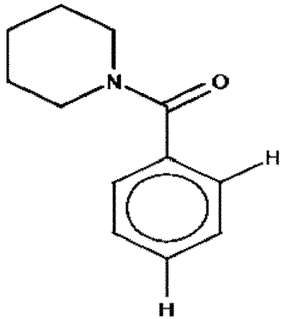 (1-benzoyl-piperidine).

In one embodiment, the inhibitor has the following formula: 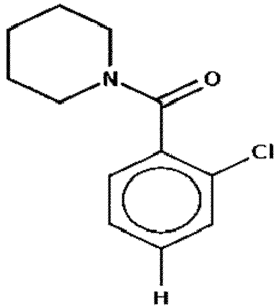 (1-(2-chlorobenzoyl)piperidine).

In one embodiment, the inhibitor has the following formula: 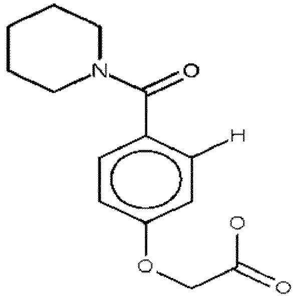 (2-[4-(piperidine-1-carbonyl)phenoxy]acetate).

In one embodiment, the inhibitor has the following formula: 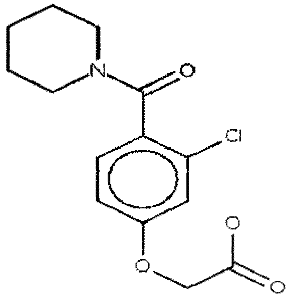 (2-[3-chloro-4-(piperidine-1-carbonyl)phenoxy]acetate).

In one embodiment, the inhibitor has the following formula: 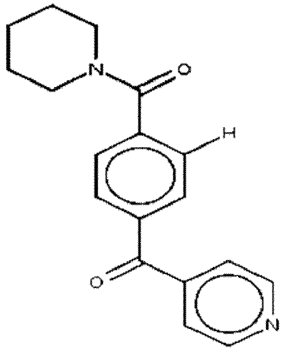 (4-[4-(piperidine-1-carbonyl)benzoyl]pyridine).

In one embodiment, the inhibitor has the following formula: 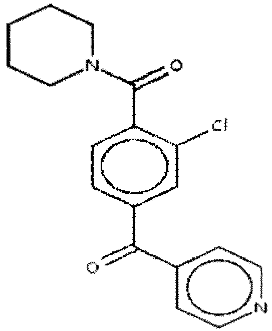 (4-[3-chloro-4-(piperidine-1-carbonyl)benzoyl]pyridine).

In one embodiment, the inhibitor has the following formula: 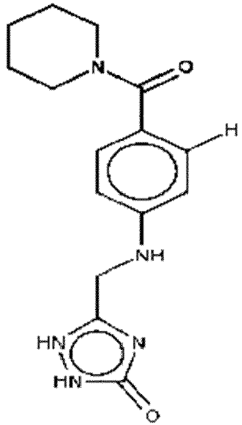 (5-({[4-(piperidine-1-carbonyl)phenyl]amino}methyl)-1,2-dihydro-1,2,4-triazol-3-one).

In one embodiment, the inhibitor has the following formula: 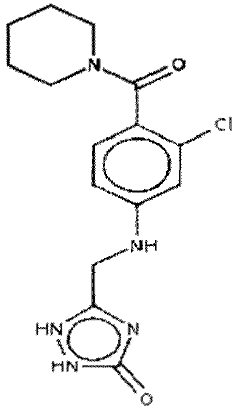 (5-({[3-chloro-4-(piperidine-1-carbonyl)phenyl]amino}methyl)-1,2-dihydro-1,2,4-triazol-3-one).

In one embodiment, the inhibitor has the following formula: 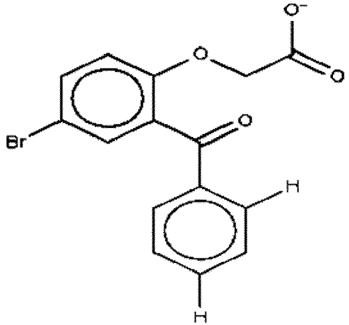 (2-(2-benzoyl-4-bromophenoxy)acetate).

In one embodiment, the inhibitor has the following formula: 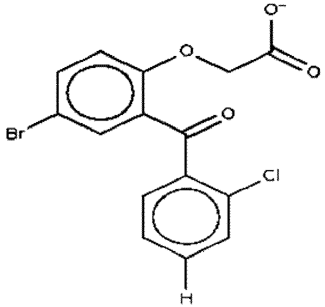 (2-[4-bromo-2-(2-chlorobenzoyl)phenoxy]acetate).

In one embodiment, the inhibitor has the following formula: 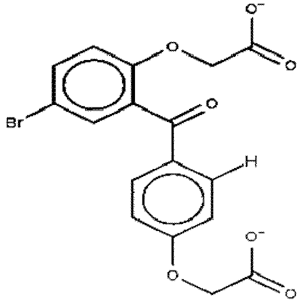 (2-{4-[5-bromo-2-(carboxylatomethoxy)benzoyl]phenoxy}acetate).

In one embodiment, the inhibitor has the following formula: 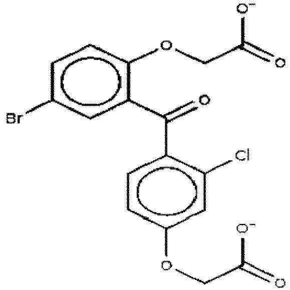 (2-{4-[5-bromo-2-(carboxylatomethoxy)benzoyl]-3-chlorophenoxy}acetate).

In one embodiment, the inhibitor has the following formula: 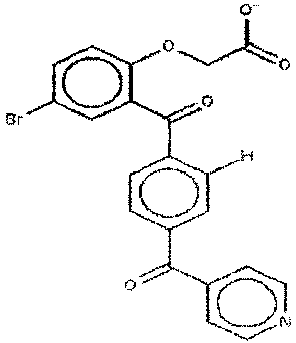 (2-{4-bromo-2-[4-(pyridine-4-carbonyl)benzoyl]phenoxy}acetate).

In one embodiment, the inhibitor has the following formula: 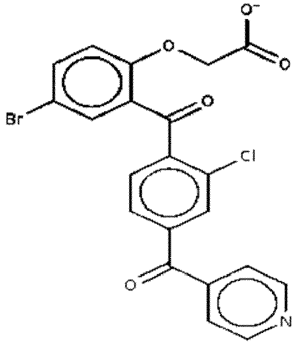 (2-{4-bromo-2-[2-chloro-4-(pyridine-4-carbonyl)benzoyl]phenoxy}acetate).

In one embodiment, the inhibitor has the following formula: 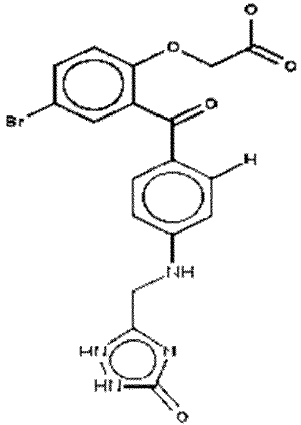 (2-[4-bromo-2-(4-{[(5-oxo-1,2-dihydro-1,2,4-triazol-3-yl) methyl]amino}benzoyl)phenoxy]acetate).

In one embodiment, the inhibitor has the following formula: 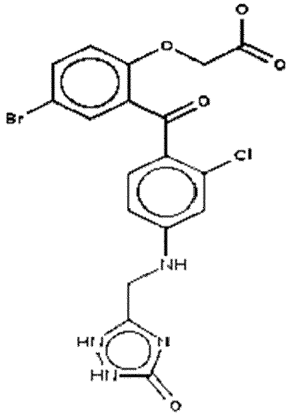 (2-[4-bromo-2-(2-chloro-4-{[(5-oxo-1,2-dihydro-1,2,4-triazol-3-yl)methyl]amino}benzoyl)phenoxy]acetate).

In one embodiment, the TRMT2A inhibitor of the invention has formula (IV): 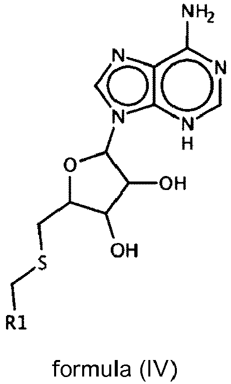 wherein
R1 is H or 2-amino-2-carboxyethyl.

In one embodiment, the inhibitor has the following formula: 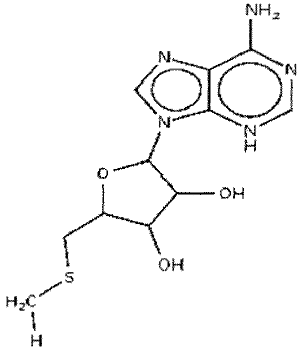 (methylthioadenosine).

In one embodiment, the inhibitor has the following formula: 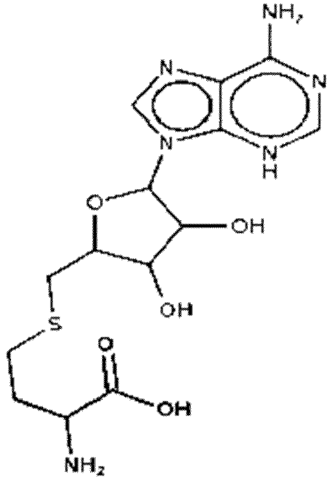 (S-adenosyl-L-homocysteine).

In one embodiment, the TRMT2A inhibitor of the invention has formula (V): 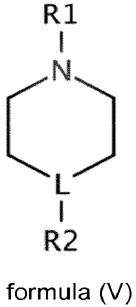 wherein
L is C or N;
R1 is (2E)-4-ethoxy-4-oxobut-2-en-2-yl or 3-(1H-pyrrol-1-yl)propanoyl; and
R2 is H or pyrimidin-2-yl.

In one embodiment, the inhibitor has the following formula: 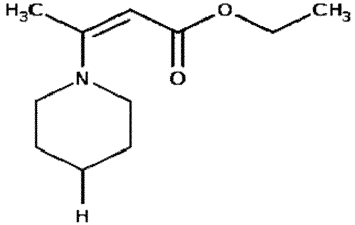 (ethyl (2Z)-3-(piperidin-1-yl)but-2-enoate).

In one embodiment, the inhibitor has the following formula: 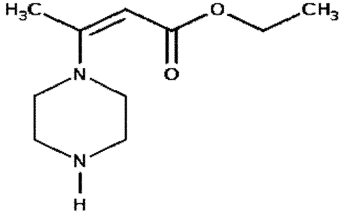 (ethyl (2Z)-3-(piperazin-1-yl)but-2-enoate).

In one embodiment, the inhibitor has the following formula: 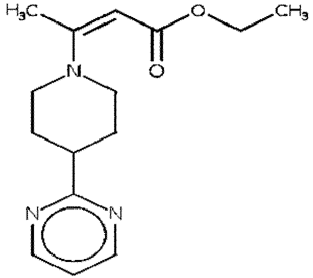 (ethyl (2Z)-3-[4-(pyrimidin-2-yl)piperidin-1-yl]but-2-enoate).

In one embodiment, the inhibitor has the following formula: 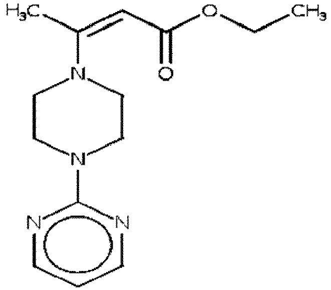 (ethyl (2Z)-3-[4-(pyrimidin-2-yl)piperazin-1-yl]but-2-enoate).

In one embodiment, the inhibitor has the following formula: 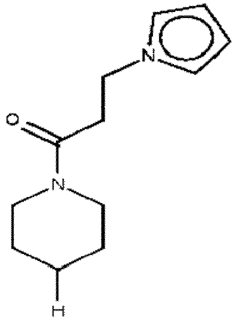 (1-(piperidin-1-yl)-3-(pyrrol-1-yl)propan-1-one).

In one embodiment, the inhibitor has the following formula: 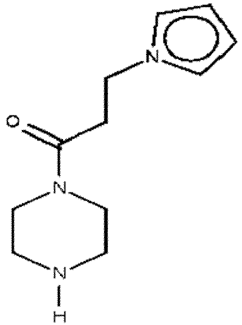 (1-(piperazin-1-yl)-3-(pyrrol-1-yl)propan-1-one).

In one embodiment, the inhibitor has the following formula: 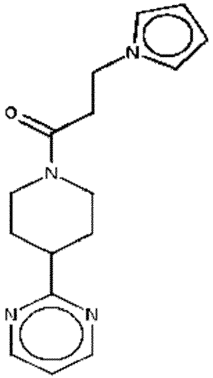 (1-[4-(pyrimidin-2-yl)piperidin-1-yl]-3-(pyrrol-1-yl)propan-1-one).

In one embodiment, the inhibitor has the following formula: 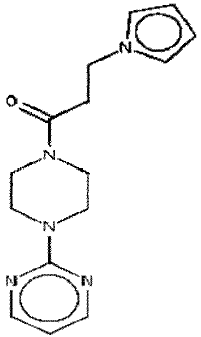 (1-[4-(pyrimidin-2-yl)piperazin-1-yl]-3-(pyrrol-1-yl)propan-1-one).

In one embodiment, the inhibitor has the following formula: 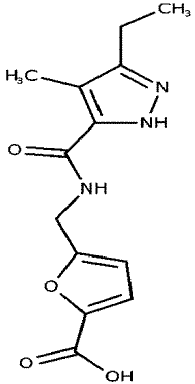 (5-{[(5-ethyl-4-methyl-2H-pyrazol-3-yl)formamido]methyl}furan-2-carboxylic acid).

In one embodiment, the inhibitor has the following formula: 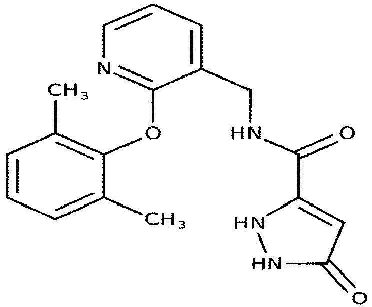 (N-{[2-(2,6-dimethylphenoxy)pyridin-3-yl]methyl}-5-oxo-1,2-dihydropyrazole-3-carboxamide).

In one embodiment, the inhibitor has the following formula: 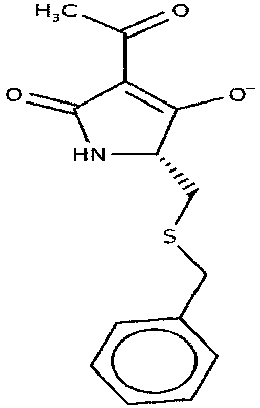 ((2R)-4-acetyl-2-[(benzylsulfanyl)methyl]-5-oxo-1,2-dihydropyrrol-3-olate).

In one embodiment, the inhibitor has the following formula: 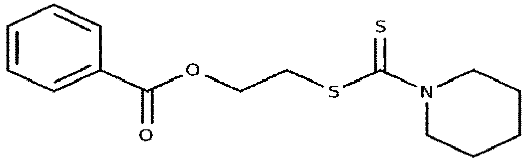 (2-(piperidine-1-carbothioylsulfanyl)ethyl benzoate).

In one embodiment, the inhibitor has the following formula: 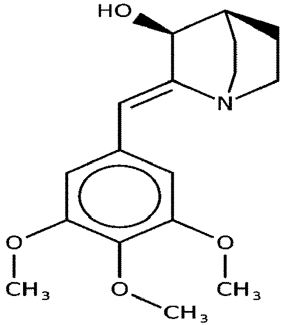 ((2Z,3S)-2-[(3,4,5-trimethoxyphenyl)methylidene]-1-azabicyclo[2.2.2]octan-3-ol).

In one embodiment, the inhibitor has the following formula: 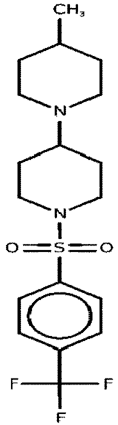 (4-methyl-1'-[4-(trifluoromethyl)benzenesulfonyl]-1,4'-bipiperidine).

In one embodiment, the inhibitor has the following formula: 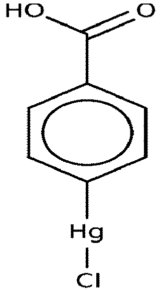 (p-chloromercuribenzoic acid).

In one embodiment, the inhibitor has the following formula: 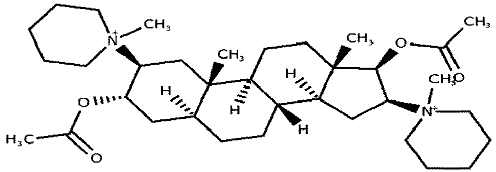 (pancuronium).

In one embodiment, the inhibitor has the following formula: 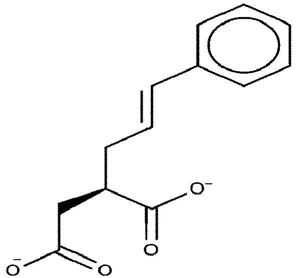 ((2R)-2-[(2E)-3-phenylprop-2-en-1-yl]butanedioate).

In one embodiment, the inhibitor has the following formula: 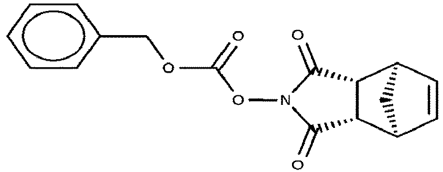 (benzyl(1R,2R,6S,7S)-3,5-dioxo-4-azatricyclo[5.2.1.0^{2,6}]dec-8-en-4-yl carbonate).

In one embodiment, the inhibitor has the following formula: 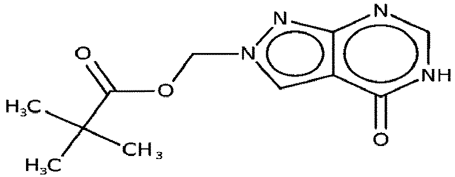 (ALLOPURINOL-2pivaloyloxymethyl).

In one embodiment, the inhibitor has the following formula: 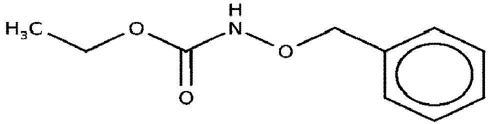 (ethyl N-(benzyloxy)carbamate).

In a particularly preferred embodiment, the inhibitor has the formula ((2R)-4-acetyl-2-[(benzylsulfanyl)methyl]-5-oxo-1,2-dihydropyrrol-3-olate). (Inhibitor 19)

In another particularly preferred embodiment, the inhibitor has the formula (4-aminobutyl)(3-aminopropyl)amine (spermidine). (Inhibitor 15)

In another particularly preferred embodiment, the inhibitor has the formula ethyl N-(benzyloxy)carbamate. (Inhibitor 25)

In another preferred embodiment, the inhibitor has the formula ethyl (2E)-3-(piperidin-1-yl)but-2-enoate. (Inhibitor 3)

In another preferred embodiment, the inhibitor has the formula 4-methyl-1'-[4-(trifluoromethyl)benzenesulfonyl]-1,4'-bipiperidine. (Inhibitor 5)

In another preferred embodiment, the inhibitor has the formula (2S)-2-aminohexanoic acid. (Inhibitor 7)

In another preferred embodiment, the inhibitor has the formula (4-carboxyphenyl)(chloro)mercury. (Inhibitor 8)

In another preferred embodiment, the inhibitor has the formula 2-amino-4-(ethylsulfanyl)butanoic acid. (Inhibitor 13)

In another preferred embodiment, the inhibitor has the formula 1-phenyl-4-(pyridine-3-carbonyl)piperazine. (Inhibitor 16)

In another preferred embodiment, the inhibitor has the formula (2R)-2-[(2E)-3-phenylprop-2-en-1-yl]butanedioate. (Inhibitor 17)

In another preferred embodiment, the inhibitor has the formula 2-{4-[5-bromo-2-(carboxymethoxy)benzoyl]phenoxy}acetate. (Inhibitor 21)

In another preferred embodiment, the inhibitor has the formula benzyl (1R,2R,6S,7S)-3,5-dioxo-4-azatricyclo[5.2.1.0^{2,6}]dec-8-en-4-yl carbonate. (Inhibitor 22)

In another preferred embodiment, the inhibitor has the formula {4-oxo-2H,4H,5H-pyrazolo[3,4-d]pyrimidin-2-yl}methyl 2,2-dimethylpropanoate. (Inhibitor 24)

**Table 1:**

| **Nº** | **IUPAC Name** |
|---|---|
| **1** | 2-(piperidine-1-carbothioylsulfanyl)ethyl benzoate |
| **2** | (2Z,3S)-2-[(3,4,5-trimethoxyphenyl)methylidene]-1-azabicyclo[2.2.2]octan-3-ol |
| **3** | ethyl (2E)-3-(piperidin-1-yl)but-2-enoate |
| **4** | 4-chloro-N-[(4-methyl-5-{[2-(piperidin-1-yl)ethyl]sulfanyl}-4H-1,2,4-triazol-3-yl)methyl]aniline |
| **5** | 4-methyl-1'-[4-(trifluoromethyl)benzenesulfonyl]-1,4'-bipiperidine |
| **6** | N-[3-chloro-2-(piperidin-1-yl)phenyl]-2-[(5-methyl-1,3,4-oxadiazol-2-yl)sulfanyl]acetamide |
| **7** | (2S)-2-aminohexanoic acid |
| **8** | (4-carboxyphenyl)(chloro)mercury |
| **9** | 1-[(1R,2S,3aS,3bR,5aS,7S,8S,9aS,9bS,11aS)-1,7-bis(acetyloxy)-9a,11a-dimethyl-2-(1-methylpiperidin-1-ium-1-yl)-hexadecahydro-1H-cyclopenta[a]phenanthren-8-yl]-1-methylpiperidin-1-ium |
| **10** | (2R,3R,4S,5S)-2-(6-amino-9H-purin-9-yl)-5-[(methylsulfanyl)methyl]oxolane-3,4-diol |
| **11** | (2S)-2-amino-4-({[(2S,3S,4R,5R)-5-(6-amino-9H-purin-9-yl)-3,4-dihydroxyoxolan-2-yl]methyl}sulfanyl)butanoic acid |
| **12** | (2R)-2-amino-4-(methylsulfanyl)butanoic acid |
| **13** | 2-amino-4-(ethylsulfanyl)butanoic acid |
| **14** | butane-1,4-diamine |
| **15** | (4-aminobutyl)(3-aminopropyl)amine |
| **16** | 1-phenyl-4-(pyridine-3-carbonyl)piperazine |
| **17** | (2R)-2-[(2E)-3-phenylprop-2-en-1-yl]butanedioate |
| **18** | 6-benzyl-4-hydroxyquinoline-2-carboxylate |
| **19** | ((2R)-4-acetyl-2-[(benzylsulfanyl)methyl]-5-oxo-1,2-dihydropyrrol-3-olate) |
| **20** | [4,6-bis(methoxycarbonyl)-5-(pyridine-2-carbonyl)pyrimidin-2-yl]sulfanide |
| **21** | 2{4-[5-bromo-2-(carboxymethoxy)benzoyl]phenoxy}acetate |
| **22** | benzyl (1R,2R,6S,7S)-3,5-dioxo-4-azatricyclo[5.2.1.0^{2,6}]dec-8-en-4-yl carbonate |
| **23** | benzyl N-(2-{[(naphthalen-1-yl)carbamoyl]oxy}ethyl)carbamate |
| **24** | {4-oxo-2H,4H,5H-pyrazolo[3,4-d]pyrimidin-2-yl}methyl 2,2-dimethylpropanoate |
| **25** | ethyl N-(benzyloxy)carbamate |
| **26** | N-[(3-chlorophenyl)methyl]-4-hydroxy-2-(pyridin-2-yl)pyrimidine-5-carboxamide |
| **27** | 5-{[(3-ethyl-4-methyl-1H-pyrazol-5-yl)formamido]methyl}furan-2-carboxylic acid |
| **28** | 1-[4-(pyrimidin-2-yl)piperazin-1-yl]-3-(1H-pyrrol-1-yl)propan-1-one |
| **29** | 1-phenyl-3-{[4-(pyridine-4-carbonyl)phenyl]amino}propan-1-one |
| **30** | N-{[2-(2,6-dimethylphenoxy)pyridin-3-yl]methyl}-5-oxo-2,5-dihydro-1H-pyrazole-3-carboxamide |
| **31** | 5-({[3-chloro-4-(piperidine-1-carbonyl)phenyl]amino}methyl)-2,3-dihydro-1H-1,2,4-triazol-3-one |
| **32** | N-benzyl-2-(4-chlorophenoxy)ethanimidamide |

### Compositions and Formulations

In another aspect, the present invention relates to pharmaceutical compositions comprising a TRMT2A inhibitor. In one embodiment, the pharmaceutical composition comprises a TRMT2A inhibitor and one or more pharmaceutically acceptable excipients. In another embodiment, the pharmaceutical composition comprises a TRMT2A inhibitor in combination with another therapeutically active agent. For example, the TRMT2A inhibitors provided herein may be combined with Tetrabenazine or its derivatives.

In one embodiment, the pharmaceutical composition is for use in the treatment and/or prevention of a polyglutamine disease. In a preferred embodiment, the pharmaceutical composition is for use in the treatment and/or prevention of Huntington's disease.

The provided compositions may be administered by any route of administration. For example, administration may occur via oral administration or via parenteral administration. Specific embodiments relate to intravenous administration, subcutaneous administration, inhalation and topical administration. Suitable formulations include for example tablets, capsules, solutions or any other suitable dosage form. The TRMT2A inhibitors may also be formulated in drug delivery systems that cross the blood-brain barrier.

### Food Products

Also provided is a food product comprising one or more of the TRMT2A inhibitors provided herein. Several of the TRMT2A inhibitors described herein are micro-nutrients which occur in certain food products . The invention provides a food product, wherein the TRMT2A inhibitor is enriched. Accordingly, these food products enable a dietary intake of therapeutic levels of TRMT2A inhibitors.

### Methods

Further provided is a method for inhibiting TRMT2A, wherein the method comprises contacting a cell *in vitro* with an inhibitor of TRMT2A.

In one embodiment, the cell is contacted with TRMT2A at a concentration of 1-1000 µM. In a further embodiment, the cell is contacted with TRMT2A at a concentration of 10-500 µM. In a further embodiment, the cell is contacted with TRMT2A at a concentration of 50-200 µM. In a further embodiment, the cell is contacted with TRMT2A at a concentration of 100 µM.

In one embodiment, the cell expresses polyglutamine containing polypeptides. Overexpression of polyglutamine may result in polyglutamine aggregation.

In one embodiment, contacting the cell with a TRMT2A inhibitor results in reduction or full inhibition of polyglutamine aggregation in comparison to untreated control cells. In one embodiment, polyglutamine aggregation is decreased by at least 10%. In a further embodiment, polyglutamine aggregation is decreased by at least 20%. In a further embodiment, polyglutamine aggregation is decreased by at least 30%. In a further embodiment, polyglutamine aggregation is decreased by at least 40%. In a further embodiment, polyglutamine aggregation is decreased by at least 50%. In a further embodiment, polyglutamine aggregation is decreased by at least 60%. In a further embodiment, polyglutamine aggregation is decreased by at least 70%.

In one embodiment, contacting the cell with a TRMT2A inhibitor results increased cell viability in comparison to untreated control cells. In one embodiment, cell viability is increased by at least 10%. In a further embodiment, cell viability is increased by at least 20%. In a further embodiment, cell viability is increased by at least 30%. In a further embodiment, cell viability is increased by at least 40%. In a further embodiment, cell viability is increased by at least 50%. In a further embodiment, cell viability is increased by at least 60%. In a further embodiment, cell viability is increased by at least 70%.

### EXAMPLES

The invention is illustrated in the following examples.

### General inhibitor screening

5000 HEK293T cells per cm² were seeded and transiently transfected with various constructs to achieve polyQ expression (polyQ103:GFP) 24 hours after plating. Four hours after post transfection, inhibitors were added to the cell culture medium (final concentrations: 100 µM). Most of the inhibitors were dissolved in either H₂O or DMSO. As a positive control, HEK293T cells with stable RNAi-mediated knockdown of TRMT2A were used. These cells were infected with Lentiviral plasmid vector (*pLKO.1-puro,* human phosphoglycerate promoter) expressing a TRMT2A-specific inverted repeat to facilitate RNAi-mediated gene silencing. Commercially available transduction particles were purchased from Sigma-Aldrich (MISSION® shRNA Lentviral Transduction Particles; NM_182984.2-**856**s1c1 and NM_182984.2-**1574**s1c1; SHC002V as scrambled shRNA control, **shK** respectively). Puromycin-resistant colonies were selected (stable integration) and decrease of TRMT2A protein abundance was determined by Western Blot analysis. A cell line with stable integration of NM_182984.2-**1574**s1c1 (short **sh1574**) displayed the strongest reduction of TRMT2A (>90%) was selected and used for further experiments.

### Transfections of HEKT293T cells

HEK293T cells were grown in Dulbecco's modified Eagle's medium (DMEM) supplemented with 10% (v/v) FBS and 1% (v/v) penicillin/streptomycin (PIS). For transfection, cells were seeded one day before the experiment at a density of 30 x 10⁴ cells per well in a 6-well-plate. The transfection mix (in relation to the total volume of cell culture medium per well) contains 1% CaCl₂ (1M), 4% ddH₂O, 5% 2 x HBSS; 0,075% (v/w) DNA. The mix was incubated for 5 minutes and added onto the cells. Four hours later the cells were treated with potential TRMT2A inhibitors (concentrations: 100 µM). 48 hours post transfection (44 hours after inhibitor administration), the cells were treated with trypsin and detached from the dishes and transferred into 1 ml of fresh DMEM-Medium.

### Example 1: Cell Death Assay (inhibitors 16-32)

A 1:1 dilution of cells in trypan blue (0.02%) was analyzed using the automated Cedex cell counter.

For verification of observed differences in cell death, the effect of few selected inhibitors was analyzed by a manual quantification of cell death. Here, a 1:100 dilution of trypsinated cells in trypan blue was analyzed in the Haematocytometer (Neubauer chamber). GFP positive cells were counted and the relative number of additionally blue cells (dead cells) was determined. Here, cell death in three biological replicates were assayed. 100 GFP-positive cells were analyzed per condition.

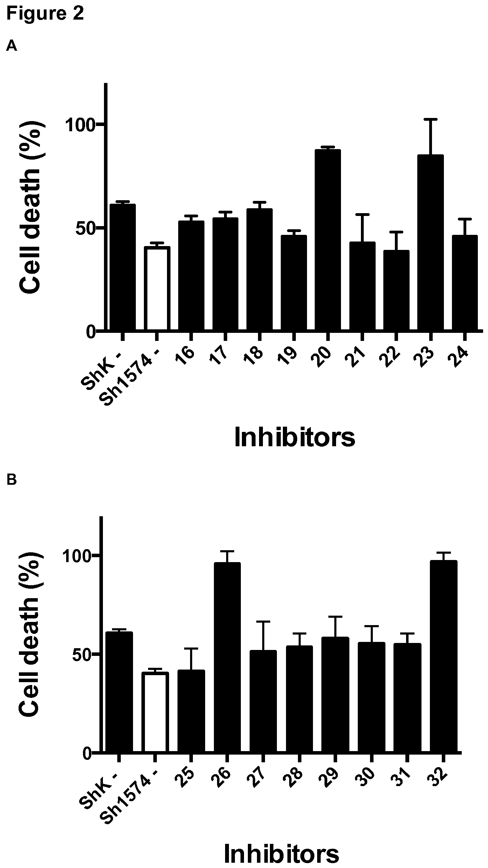

Compared were **shK** cells with overexpression of polyQ in presence or absence of tested inhibitors. The percentage of dead cells after 48 hours was decreased upon treatment with different TRMT2A inhibitors (Figure 1 and Figure 2). **Sh1574** cells served as internal control. Since these cells do not have significant amounts of the TRMT2A protein, a specific TRMT2A inhibitor should not display any additional protective effects towards polyQ-induced toxicity.

### Example 2: Western Blot

PolyQ-induced cell death is tightly linked to the expression levels of polyQ-peptides. Consequently, we performed Western blot analysis to detect soluble polyQ-abundance in presence or absence of inhibitor. In case the inhibitor would somehow influence polyQ expression, we would expect reduced levels of soluble polyQ-peptides. To rule out this possibility, abundance of soluble polyQ peptides derived from cells in presence or absence of a respective inhibitor was compared.

For this kind of analysis, six-well plates were used to obtain enough material for subsequent analysis. In brief, cells were washed in PBS and then mechanically detached from the bottom of the plate in 100 µl RIPA buffer. Samples were centrifuged for 30 min at 13,300 rpm at 4°C and supernatants were collected (RIPA soluble fraction). The pellet (RIPA insoluble fraction) was used for Dot Blot analysis (see below, Dot Blot). Protein concentrations of the soluble fractions were measured using the Bio-Rad DC Protein Assay (according to the manufacturers instructions) and Tecan® multimode microplate reader.

In brief: To determine the protein concentration of the RIPA soluble fraction, the Bradford protein assay (DC Protein assay, Bio-Rad) was used according to the manufacturer's instructions (mixture A' was generated by mixing 24.5 µl of solution A (alkaline copper tartrate solution for colorimetric assays) and 5 µl of solution S (surfactant solution for colorimetric assays).

### Western blotting:

For Western blot analysis of the RIPA soluble fraction, 30 µg protein samples were diluted in loading buffer (5x Laemmli) and boiled (95 °C, 5 min) before loading onto a polyacrylamide gel (12%) and subsequent SDS-PAGE (run at 100 V for 90 min with running buffer). Resolved proteins were transferred onto nitrocellulose membrane (225 mA per gel for 60 min with transfer buffer using a semi dry blotter). The membranes were then blocked with skim milk (5% in TBS-T for 60 min) followed by incubation with the primary antibody at 4°C overnight (in 0.5% skim milk in TBS-T). The primary antibody (mouse anti-Polyglutamine; Milipore MAB1574 or mouse monoclonal anti-Huntingtin; DSHB MW1 or mouse monoclonal anti-GFP; Roche 11814460001; all 1:1000) was diluted in blocking solution and incubated under agitation overnight at 4°C. Membranes were washed three times for 10 minutes in TBS-T and incubated with the secondary antibody (ECL anti mouse IGG, hrp-linked from sheep; GE Healthcare, NXA931V; 1:3000) for at least one hour under agitation at room temperature or at 4°C overnight.
Subsequently, the membranes were washed three times in TBS-T for 10 minutes and the chemiluminescence signal was detected using the Super Signal® West Femto Maximum Sensitivity Substrate (Thermo Scientific) detection kit according to manufacturer's instructions. Chemiluminescence signals were visualized using the Alliance UVItec system (Biometra) and processed using Image J software.

All tested compounds did not cause a reduction in polyQ-abundance in the soluble fraction.

### Dot Blot (inhibitors 1-15 and selected/positive ones 16-32):

The pellet (RIPA insoluble fraction) was washed twice in 100 µl RIPA-buffer, re-suspended in 100 µl Urea buffer, incubated for 60 minutes at 4°C under rotation and subsequently centrifuged at 4°C, 9,000 g for 30 min. The supernatant was applied for further analysis and the urea insoluble pellet was discarded. The urea soluble fraction were homogenized for 10 minutes in the ultrasonic bath (VWR, Germany).
The insoluble aggregates were relatively quantified via the filter retardation assay (FRA). RIPA insoluble pellet was dissolved in urea buffer (30 mM Tris Buffer, 7 M Urea, 2 M Thiourea, 4% CHAPS pH - 8.5; 10 µl/fly head) by sonication (incubation in Ultrasonic Cleaner bath, VWR) for 30 minutes followed by centrifugation > 15,000 x g for 30 minutes at 4°C. The supernatant was transferred to an Eppendorf tube and considered the (RIPA-) insoluble fraction. It is impossible to determine the protein concentration of the RIPA-insoluble fraction. Thus, we used the amount of protein of the RIPA-soluble fraction as reference. Relative to the total protein concentration in the RIPA soluble fraction, we loaded a volume matching 90 µg of protein. The volume was adjusted to 50 µl by addition of RIPA buffer supplemented with 7 µl dot blot buffer (0.5 M Tris pH-6.8, 0.4% SDS, 20% Glycerol, 0.2 M DTT). The samples were transferred to a 0.2 µM nitrocellulose membrane (Protran®BA 83, Whatman, pre-incubated in 1% SDS in TBS) by sucking the probes through the membrane using a vacuum pump attached to a dot blot chamber (Camlab, UK). The membrane was washed several times with TBS-T and blocked for 1 hour using 5% skim milk in TBS-T. Afterwards the membrane was treated as described for Western blotting using respective primary and secondary antibodies.

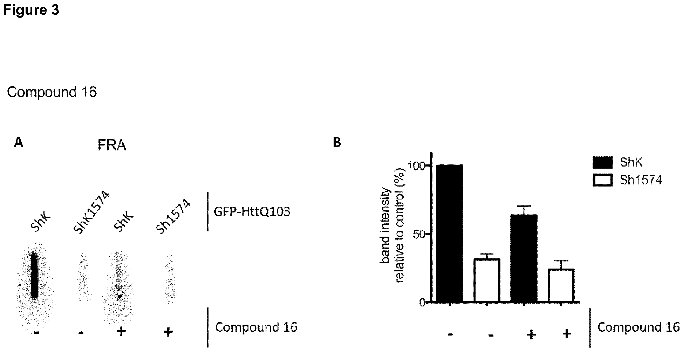

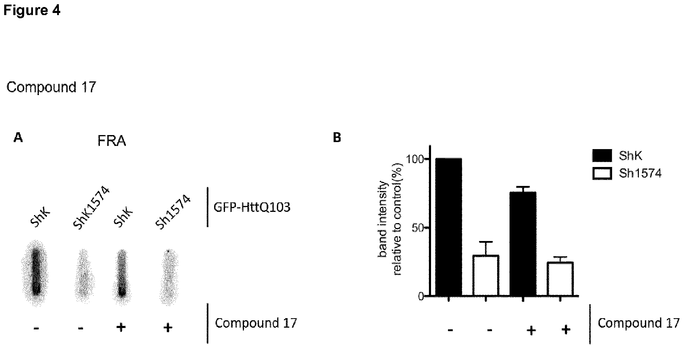

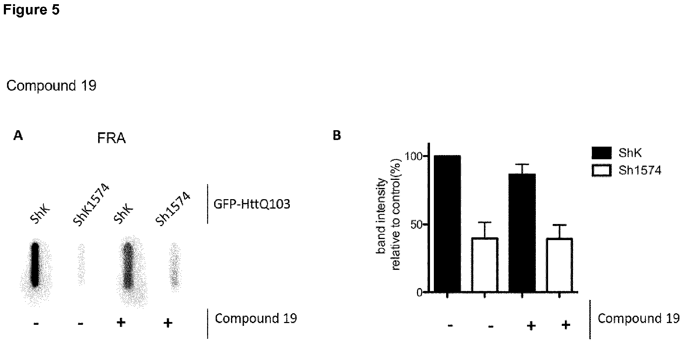

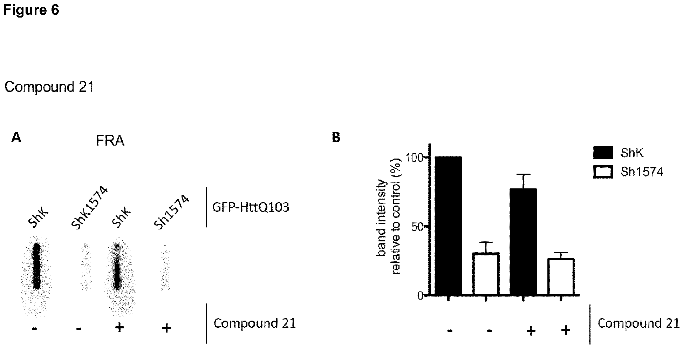

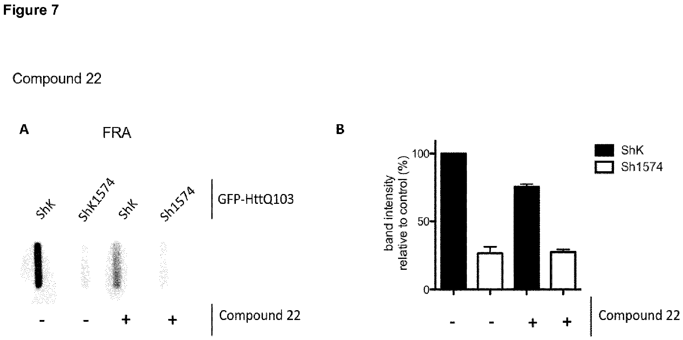

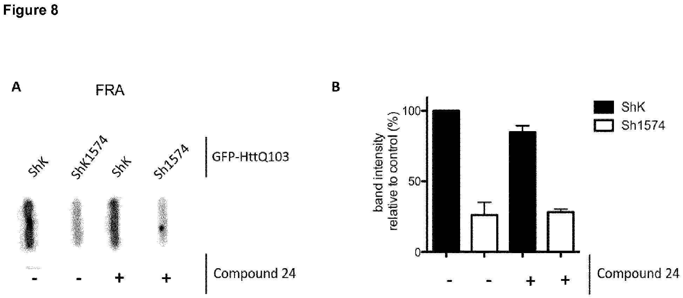

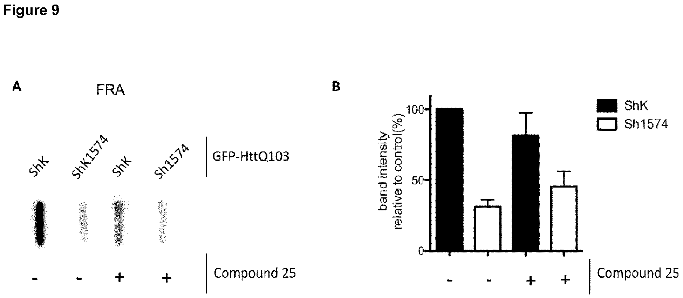

As shown in Figures 1 and 3-9, the band intensity was decreased in cells treated with TRMT2A inhibitors compared to untreated control indicating less polyQ aggregation.

### Example 3: Structure determination of the TRMT2A RNA-binding domain by X-ray crystallography

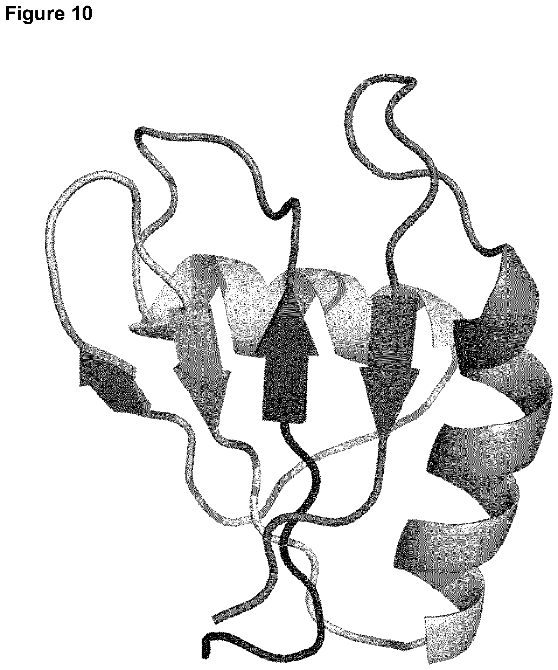

The sequence encoding the TRMT2A RNA-binding domain reaching from amino acid 69 to 147 was cloned into the pOPINS3C vector, resulting in the expression of an insert with the sequence (69-147) gp TSEIFKLELQ NVPRHASFSD VRRFLGRFGL QPHKTKLFGQ PPCAFVTFRS AAERDKALRV LHGALWKGRP LSVRLARPK (small letters indicate amino acids from rhinovirus 3C protease cleavage site). 6 liter of *E.coli Rosetta* cell culture (IPTG induction overnight at 18°C) were centrifuged and the pellet was resuspended in 50 ml lysis buffer (500 mM NaCl; 50 mM HEPES, pH 8,5; 2% Glycerol; 20 mM Imidazole; 0,5% Tween; protease inhibitor), followed by cell lysis via sonication. The supernatant was cleared by centrifugation for 40 minutes with 17000g at 4°C and filtered with a 2.7 µm filter unit. Using an Äkta Purifier system, the supernatant was loaded onto a HisTrap FF column (5 mL) column, washed with 5 column volumes of high salt buffer (2 M NaCl; 50 mM HEPES, pH 8,5; 20 mM Imidazole) and eluted via gradient elution using lysis and elution buffer (500 mM NaCl; 50 mM HEPES, pH 8,5; 500 mM Imidazole). Subsequently, the protein containing fractions were joined and the rhinovirus 3C protease added to cleave between the tag and the TRMT2A fragment. Protein was dialysed in dialysis buffer (500 mM NaCl; 50 mM HEPES, pH 7,5; 1 mM DTT) at 4°C overnight. Then the protein sample was loaded onto a HisTrap FF column (GE Healthcare) and the flow through containing the TRMT2A protein without tag was collected. Protein was concentrated to 5 mL using a 3K concentrator (Millipore) while 1 mL 5M NaCl was added to prevent precipitation. Subsequently, protein was loaded onto a Superdex75 column (GE Healthcare) and fractionated in SEC buffer (500 mM NaCl; 50 mM HEPES, pH 7,5). Protein samples showed purity of ≥ 95% and were concentrated to 8 mg/mL with a 3K concentrator. Crystallization trials were performed with hanging drop method. Trials with protein alone was performed at 20°C with 0.1 M NaAc pH 4.5; 26% PEG 8000; 0.2 M Lithium sulphate. After 7-10 days small crystals appeared (Figure 10).

### BIBLIOGRAPHY

Bates, G. P. (2005). "History of genetic disease: the molecular genetics of Huntington disease - a history." Nat Rev Genet 6(10): 766-773.
Beebe, K., M. Mock, E. Merriman and P. Schimmel (2008). "Distinct domains of tRNA synthetase recognize the same base pair." Nature 451(7174): 90.
Brogueira Rodrigues, F. and E. Wild (2018). "Huntington's Disease Clinical Trials Corner: February 2018." Journal of Huntington's Disease.
Colpo, G., E. Stimming, N. Rocha and A. Teixeira (2017). "Promises and pitfalls of immune-based strategies for Huntington's disease." Neural Regeneration Research 12(9): 1422-1425.
Colpo, G. D., E. F. Stimming, N. P. Rocha and A. L. Teixeira (2017). "Promises and pitfalls of immune-based strategies for Huntington's disease." Neural regeneration research 12(9): 1422.
Dunin-Horkawicz, S., A. Czerwoniec, M. J. Gajda, M. Feder, H. Grosjean and J. M. Bujnicki (2006). "MODOMICS: a database of RNA modification pathways." Nucleic acids research 34(suppl_1): D145-D149.
Fan, H.-C., L.-I. Ho, C.-S. Chi, S.-J. Chen, G.-S. Peng, T.-M. Chan, S.-Z. Lin and H.-J. Harn (2014). "Polyglutamine (PolyQ) Diseases: Genetics to Treatments." Cell Transplantation 23(4-5): 441-458.
Guo, M. and P. Schimmel (2013). "Essential nontranslational functions of tRNA synthetases." Nature chemical biology 9(3): 145.
Horiuchi, K. Y., M. R. Harpel, L. Shen, Y. Luo, K. C. Rogers and R. A. Copeland (2001). "Mechanistic studies of reaction coupling in Glu-tRNAGln amidotransferase." Biochemistry 40(21): 6450-6457.
Ibba, M., A. W. Curnow and D. Söll (1997). "Aminoacyl-tRNA synthesis: divergent routes to a common goal." Trends in biochemical sciences 22(2): 39-42.
Labbadia, J. and R. I. Morimoto (2013). "Huntington's disease: underlying molecular mechanisms and emerging concepts." Trends in Biochemical Sciences 38(8): 378-385.
Landles, C. and G. P. Bates (2004). "Huntingtin and the molecular pathogenesis of Huntington's disease." Fourth in Molecular Medicine Review Series 5(10): 958-963.
Menon, R. P., S. Nethisinghe, S. Faggiano, T. Vannocci, H. Rezaei, S. Pemble, M. G. Sweeney, N. W. Wood, M. B. Davis and A. Pastore (2013). "The role of interruptions in polyQ in the pathology of SCA1." PLoS genetics 9(7): e1003648.
Nordlund, M. E., J. M. JOHANSSON, U. von Pawel-Rammingen and A. S. BYSTROeM (2000). "Identification of the TRM2 gene encoding the tRNA (m 5 U 54) methyltransferase of Saccharomyces cerevisiae." Rna 6(6): 844-860.
Pringsheim, T., K. Wiltshire, L. Day, J. Dykeman, T. Steeves and N. Jette (2012). "The incidence and prevalence of Huntington's disease: A systematic review and meta-analysis." Movement Disorders 27(9): 1083-1091.
Rodriguez-Hernandez, A., H. Bhaskaran, A. Hadd and J. J. Perona (2010). "Synthesis of Glu-tRNAGln by engineered and natural aminoacyl-tRNA synthetases." Biochemistry 49(31): 6727-6736.
Schön, A., C. G. Kannangara, S. Cough and D. SÖll (1988). "Protein biosynthesis in organelles requires misaminoacylation of tRNA." Nature 331(6152): 187.
Schön, A. and D. Söll (1988). "tRNA specificity of a mischarging aminoacyl-tRNA synthetase: glutamyl-tRNA synthetase from barley chloroplasts." FEBS Letters 228(2): 241-244.
Sharma, S., J. Yang, S. Düttmann, P. Watzinger, P. Kötter and K.-D. Entian (2013). "Identification of novel methyltransferases, Bmt5 and Bmt6, responsible for the m3U methylations of 25S rRNA in Saccharomyces cerevisiae." Nucleic acids research 42(5): 3246-3260.
Sheppard, K., J. Yuan, M. J. Hohn, B. Jester, K. M. Devine and D. Söll (2008). "From one amino acid to another: tRNA-dependent amino acid biosynthesis." Nucleic acids research 36(6): 1813-1825.
Southwell, A. L., N. H. Skotte, C. F. Bennett and M. R. Hayden (2012). "Antisense oligonucleotide therapeutics for inherited neurodegenerative diseases." Trends in Molecular Medicine 18(11): 634-643.
Takeuchi, T. and Y. Nagai (2017). "Protein Misfolding and Aggregation as a Therapeutic Target for Polyglutamine Diseases." Brain Sciences 7(10): 128.
Towns, W. L. and T. J. Begley (2012). "Transfer RNA methytransferases and their corresponding modifications in budding yeast and humans: activities, predications, and potential roles in human health." DNA and cell biology 31(4): 434-454.
Zoghbi, H. Y. and H. T. Orr (2000). "Glutamine repeats and neurodegeneration." Annu Rev Neurosci 23: 217-247.

## Claims

1. An inhibitor of the enzyme TRMT2A for use in therapy.

2. An inhibitor of the enzyme TRMT2A for use in treating and/or preventing a polyglutamine disease in a subject.

3. The inhibitor for the use of claim 2, wherein the polyglutamine disease is Huntington's disease, a spinocerebellar ataxia, dentatorubral pallidoluysian atrophy or spinal and bulbar muscular atrophy.

4. The inhibitor for the use of claim 3, wherein the polyglutamine disease is Huntington's disease.

5. The inhibitor for the use of any one of claims 1-4, wherein the inhibitor has formula (I) 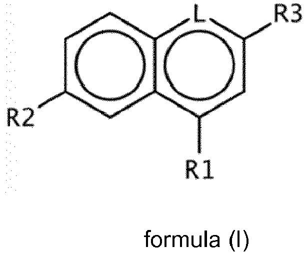 wherein
L is C or N;
R1 is OH or [(2-{[(benzyloxy)carbonyl] amino}ethoxy) carbonyl]amino;
R2 is H or benzyl; and
R3 is H or CO₂.

6. The inhibitor for the use of any one of claims 1-4, wherein the inhibitor has formula (II) 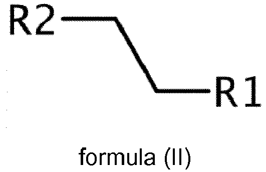 wherein
R1 is H, CH₃, NH₂, SCH₃, ethylsulfanyl or (3-aminopropyl)amino; and
R2 is amino(carboxy)methyl or CH₂CHNH₂R3, wherein R3 is H or COOH.

7. The inhibitor for the use of any one of claims 1-4, wherein the inhibitor has formula (III) 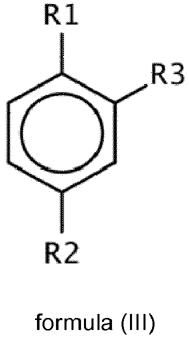 wherein
R1 is selected from the group consisting of piperidine-1-carbonyl, 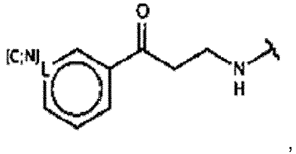 5-bromo-2-(carboxylatomethoxy)benzoyl, and 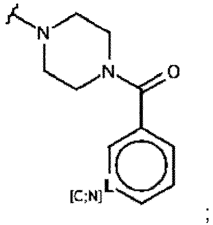
R2 is selected from the group consisting of hydrogen, carboxylatomethoxy, pyridine-4-carbonyl, and [(5-oxo-2,5-dihydro-1H-1,2,4-triazol-3-yl)methyl]amino;
R3 is selected from the group consisting of hydrogen, and Cl.

8. The inhibitor for the use of any one of claims 1-4, wherein the inhibitor has formula (IV) 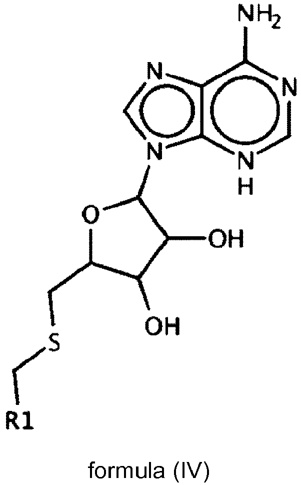 wherein R1 is H or 2-amino-2-carboxyethyl.

9. The inhibitor for the use of any one of claims 1-4, wherein the inhibitor has formula (V) 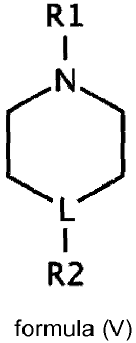 wherein
L is C or N;
R1 is (2E)-4-ethoxy-4-oxobut-2-en-2-yl or 3-(1H-pyrrol-1-yl)propanoyl; and
R2 is H or pyrimidin-2-yl.

10. The inhibitor for the use of any one of claims 1-4, wherein the inhibitor has one of the following formulas: 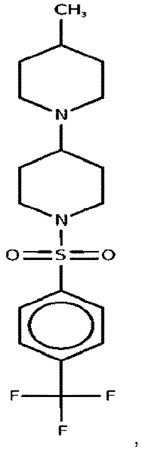 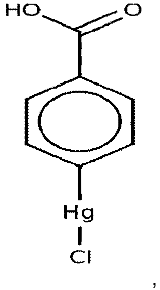 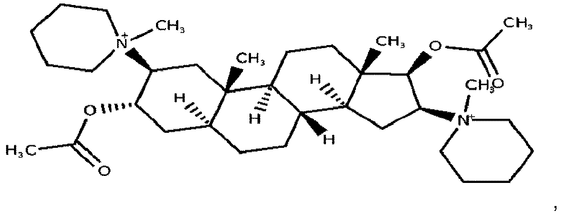 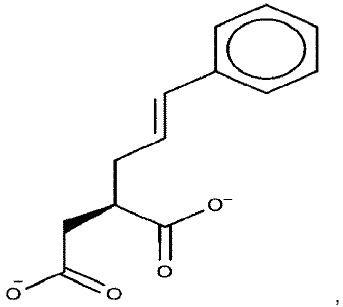 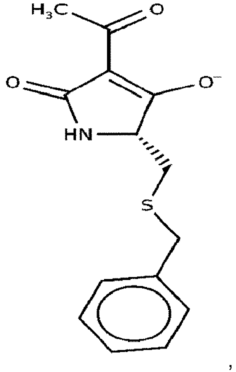 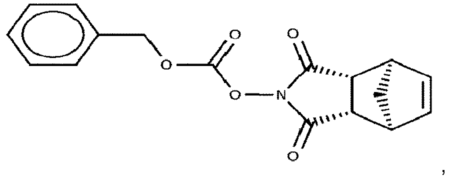 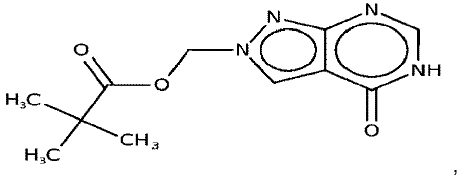 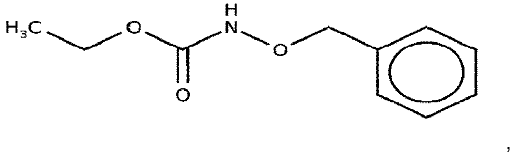 or

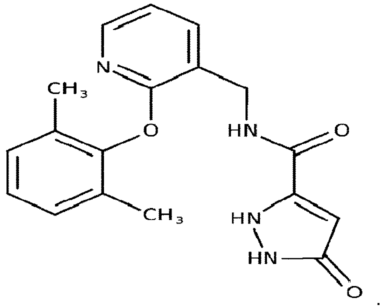

11. The inhibitor for the use any one of claims 1-10, wherein the inhibitor is administered via oral administration or via parenteral administration.

12. A pharmaceutical composition comprising a TRMT2A inhibitor and a pharmaceutically acceptable excipient.

13. The composition of claim 13, wherein the composition further comprises one or more therapeutically active agents.

14. A food product comprising a TRMT2A inhibitor.

15. A method for inhibiting TRMT2A, wherein the method comprises contacting a cell *in vitro* with an inhibitor of TRMT2A, optionally wherein the cell is contacted with the inhibitor of TRMT2A at a concentration of 10-1000 µM.
